# EUROPEAN PATENT SPECIFICATION

(11) **EP 4 144 292 B1**
(45) Date of publication and mention of the grant of the patent: **04.03.2026**
(21) Application number: 21194650.4
(22) Date of filing: 02.09.2021
(51) Int. Cl.: A61B 5/145, G16H 50/20

(54) **PROCESS AND ANALYTE MONITOR FOR ESTIMATING A QUANTITY IN MATHEMATICAL RELATIONSHIP WITH AN ANALYTE CONCENTRATION LEVEL IN A TARGET**
VERFAHREN UND ANALYTMONITOR ZUR BESTIMMUNG EINER MENGE IM MATHEMATISCHEN VERHÄLTNIS MIT EINEM ANALYTKONZENTRATIONSPEGEL IN EINEM ZIEL
PROCÉDÉ ET MONITEUR D'ANALYTE POUR L'ESTIMATION D'UNE QUANTITÉ EN RELATION MATHÉMATIQUE AVEC UN NIVEAU DE CONCENTRATION D'ANALYTE DANS UNE CIBLE

(43) Date of publication of application: 08.03.2023
(73) Proprietor: Eclypia, 38000 Grenoble (FR)
(72) Inventor: GALLEGOS, Alexandre, 75002 Paris (FR); BLANC, Romain, 38000 Grenoble (FR)
(74) Representative: Fidal Innovation

(56) References cited:
- WO-A1-2020/110222
- US-A1- 2020 193 326
- REN ZHONG ET AL: "Effects of multiple factors on the photoacoustic detection of glucose based on artificial neural network", PROCEEDINGS OF SPIE; [PROCEEDINGS OF SPIE; ISSN 0277-786X; VOL. 8615], SPIE, 1000 20TH ST. BELLINGHAM WA 98225-6705 USA, vol. 10820, 23 October 2018 (2018-10-23), pages 108201E - 108201E, XP060112615, ISBN: 978-1-5106-2099-5, DOI: 10.1117/12.2500278

## Description

### FIELD OF THE INVENTION

The present invention relates to a process for estimating an analyte concentration level, for example a process for estimating a blood sugar level, and to an analyte monitor and a computer program.

### TECHNOLOGICAL BACKGROUND

425 million people suffer from diabetes, including type 1 and type 2, worldwide. If the blood glucose (blood sugar) level of diabetic patients is not maintained, they may have serious complications, such as cardiovascular disease, kidney disease, and diabetic foot.

The first step in diabetes care is to monitor the patient's blood glucose level regularly, and if possible 24 hours a day, so as to address high blood glucose levels promptly.

The blood glucose levels may be determined from blood samples obtained invasively. Obtaining the blood by pricking a finger can be very inconvenient for a diabetic patient who needs to have their blood sugar measured several times a day, and there is further a risk of infection. Therefore, non-invasive blood glucose measurement methods are preferred when possible.

Various methods have been proposed to date for non-invasively measuring a constituent concentration in a living body, and in particular, methods based on percutaneous irradiation of electromagnetic wave. In these methods, an interaction (such as absorption or scattering) between a target blood constituent, for example, glucose in the case of a blood sugar level, and the electromagnetic waves having a particular wavelength is exploited.

A beam of light irradiates a selected part of the human body, such as a finger, the forearm, tongue, lip, thigh or abdomen and so on.

The light that is transmitted through, reflected or scattered out of the skin contains information about the composition of the irradiated tissue. This light can as a consequence be received by optical detectors and analyzed to determine the concentrations of certain analytes, such as glucose, oxygen or hemoglobin.

Photoacoustic (PA) methods don't analyze a transmitted or reflected light beam but are rather based on the detection of a thermal wave that is generated in response to the irradiation of the living body with the light beam.

When the living body is irradiated with a certain amount of electromagnetic wave, if the wavelength of the electromagnetic wave is appropriate, the electromagnetic wave can be absorbed by a target molecule contained in the living body. The optical absorption is followed by thermal expansion and thermal de-excitation.

If the irradiating light is intensity-modulated, a thermal wave is generated in the living body, and possibly an acoustic wave in the gas surrounding the living body. The thermal wave or the acoustic wave can be sensed with an appropriate sensor, such as a thermal sensor or an electroacoustic sensor. The sensed signal correlates with the target molecule concentration to be measured.

In the human body, glucose is dissolved in fluids and blood, which both consist mainly of water.

Water significantly absorbs lights having wavelengths longer than 1 µm. In a concentration ranging from 50 to 100 mg/dL (2.8 to 5.6 mM) which is the blood sugar level of a healthy subject, water may have an absorbance up to 1000 times larger than the absorbance of glucose. Consequently, the thermal wave and the acoustic wave may also correlate with other concentrations (such as water) than the target molecule concentration (such as glucose).

So far, most spectroscopic measurements focused on the near-infrared (NIR) region because NIR light can penetrate up to several millimeters into human tissue and reach blood vessels. Unfortunately, however, the glucose absorption in the NIR region is weak and NIR light interferes strongly with other blood and tissue components. As a consequence, accuracy issues need to be solved before future clinical applications can be envisaged.

In contrast, the mid-IR region benefits from strong glucose absorption but at the price of strong water absorption. Generally, background absorption caused by water in the mid-infrared region is too high to permit accurate non-invasive blood glucose measurements.

At wavelengths shorter than 1500 nm, the absorption of light by glucose is again overshadowed by background absorption in water or the skin. As a consequence, changes in the signal detected due to blood glucose concentration changes may be masked by water absorbance and/or the changes in this absorbance.

Whatever the wavelength of the irradiating beam, one understands that the extraction of the part of the signal specific to the target molecule is a crucial point.

Besides, numerous parameters can strongly affect PA generation and propagation, including optical parameters as well as other physiological parameters such as the expansion coefficient, specific of heat, acoustic velocity, acoustic impedance, acoustic attenuation and thermal conductivity of the irradiated region, volumetric changes in blood, and of course, pressure, moisture and temperature.

WO 2020/110222 relates to a device and method to measure a concentration, notably of glycemia, comprising a first step of irradiating a target object with a first irradiation device emitting light at a first wavelength (absorbed by the component of interest) and with a second irradiation device emitting light at a second wavelength (absorbed differently from the first wavelength by the component of interest) with a first intensity E1 (simultaneously or not); a first step of detecting the pressure wave generated following the first irradiation; a second step of irradiating a target object with the first irradiation device and the second irradiation device emitting light at the second wavelength with a second intensity (simultaneously or not) and second step of detecting the pressure wave generated following the second irradiation. For the first irradiation, the temperature of the medium is different from that of the second irradiation because the intensities used for the two irradiations are not the same. Following these steps, a correction coefficient for environmental factors (water concentration, performance of the pressure sensor) is determined by a correction unit, on the basis of the results of the two irradiation steps. In particular, an algorithm can be obtained by learning for the determination of this correction coefficient. Indeed, the irradiation with the second wavelength causes a shift in the absorption spectrum of the target which is related to the intensity of the incident light. The intensities of the signals detected at the first and at the second detection step are corrected by means of this correction coefficient. A temperature information acquisition unit also determines, for example via an algorithm obtained by learning, information on the temperature in the target on the basis of the results of the second irradiation. Finally, the glucose concentration can be determined by means of an algorithm obtained by machine learning, receiving as input the corrected intensity of the second detection step and the information on the temperature in the target.

US 2020/193,326 ("D2") relates to a method for training a model implemented in the context of monitoring a physiological parameter, for example glycemia. The target is irradiated with radio waves of millimeter wavelength and frequencies preferably in the 122 GHz -126 GHz range, which the inventors have found correspond to a shallower penetration depth than lower frequencies and to a better focusing of the beam, and allow a reduction of the size of the antennas, and the signal reflected by the target is then detected. The Doppler effect is then used to isolate the fraction of the detected signal corresponding to the blood flow, fraction from which the glycemia is then deduced. In one embodiment, a model can be trained to allow a blood glucose value to be determined from the magnitudes and/or phases of the components of the sensed signal. Training data can be obtained using reference samples whose glucose concentration is constant and known, or varies in a known way. Different models can be obtained by learning, corresponding to ranges of different physiological situations. Models can be improved as new data is collected.

Ren Zhong et al. "Effects of multiple factors on the photoacoustic detection of glucose based on artificial neural network", proceedings of SPIE, vol. 10820, 23 october 2018, p. 108201E, aims to investigate the effects of factors influencing the photoacoustic signal in the context of glucose detection by this technique. A forward propagation neural network is trained using a data set comprising analyte concentration, irradiation energy, temperature and flow velocity of the fluid in which the analyte concentration is to be measured, as well as the amplitude of the corresponding photoacoustic signal. After training, the neural network is able to estimate the analyte concentration from the other parameters and the amplitude of the photoacoustic signal.

US20200253513A1 describes a method to increase the sensitivity of a blood glucose sensor based on the photoacoustic detection. This document describes in particular a method of learning an algorithm for estimating the concentration of an analyte from acoustic signals.

The method includes:
- a step comprising :
   a) obtaining acoustic signals with a sensor of a first measuring device (such as a blood glucose sensor based on photoacoustic detection), and
   b) simultaneously obtaining analyte concentrations using a second reference measuring device (such as a conventional blood glucose sensor) in order to form a learning set, the first and second measuring devices being different from each other;
- a supervised learning step of an algorithm from the learning set;
- a step of estimating the concentration of the analyte from the data from the first measuring device and from the algorithm.

US2020352484A1, filed as a continuation in part of US20200253513A1, further discloses a calibration step can be performed before the measurement, so as to improve the accuracy of the measurement. The calibration step can comprise thermal monitoring, measuring the background absorption or electric impedance spectroscopy.

In all cases, the learning set comprises measurements in which the glucose concentration and all the parameters can vary at the same time. That is to say, glucose is placed at the same level as the other influencing parameters, even though its contribution to the detected signal may be rather low.

Furthermore, due to the number of influencing parameters, estimating a model is rather complex in this case.

As a consequence, the blood glucose prediction capacity of an algorithm trained with such a learning set is highly dependent on the quality of the training set.

The invention aims at proposing a process for estimating a quantity in mathematical relationship with an analyte concentration comprising a step of learning an algorithm which predicts the changes in a particular analyte concentration more accurately than prior art processes.

### SUMMARY OF THE INVENTION

The invention relates to a process for estimating a quantity in mathematical relationship with an analyte concentration level at an analysis date t in a target, with an analysis apparatus comprising :
- a wave emitter emitting an electromagnetic wave or an acoustic wave,
- a main sensor configured to sense directly or indirectly a wave being generated in the target in response to an irradiation of the target with the wave emitter,
- at least one additional sensor configured to measure at least one additional parameter influencing a signal sensed by the main sensor,
- at least one processor.

The process comprises:
a) providing a set of models comprising :
   - an environmental model receiving as input at least one additional datum at an analysis date t from the at least one additional sensor and at least one real main sensor datum from the main sensor at a prior date t' earlier than the analysis date t, and providing at least one expected main sensor datum at the analysis date t that estimates at least one datum which would have been obtained with the main sensor at the analysis date t if the analyte concentration level had remained the same at the analysis date t as at the prior date t',
   - and a principal model receiving said at least one expected main sensor datum at the analysis date t from said environmental model and at least one real main sensor datum from the main sensor at the analysis date t, and estimating a quantity in mathematical relationship with an analyte concentration level at the analysis date t ;
b) triggering the irradiation of the target with the wave emitter at a prior date t' earlier than the analysis date t in order to generate a first wave in the target in response to the irradiation, and directly or indirectly detecting this first generated wave with the main sensor in order to acquire at least one real main sensor datum at the prior date t';
c) triggering the irradiation of the target with the wave emitter at the analysis date t in order to generate a second wave in the target in response to the irradiation, directly or indirectly detecting this second generated wave with the main sensor in order to acquire at least one real main sensor datum at the analysis date t, and measuring at least one additional parameter with at least one additional sensor in order to acquire at least one additional datum at the analysis date t with at least one additional sensor ;
d) calculating at least one expected main sensor datum at the analysis date t based on the environmental model, on said at least one additional datum at the analysis date t and on said at least one real main sensor datum at the prior date t' with a processor implementing the environmental model ;
e) calculating a quantity in mathematical relationship with an analyte concentration level at the analysis date t based on the principal model, said at least one real main sensor datum at the analysis date t and the calculated at least one expected main sensor datum at the analysis date t with a processor implementing the principal model.

The signal sensed by the main sensor is in general correlated with the analyte concentration in the target, but many additional parameters influence at the same time the signal sensed by the main sensor. **In** consequence, it is difficult to extract the analyte-specific part of the signal sensed by the main sensor in order to be able to estimate accurately a quantity in mathematical relationship with the analyte concentration level in a target such as this concentration itself, or a change of this concentration, or a rate of change of this concentration.

The invention consists in using an environmental model trained to be able to estimate at least one expected main sensor datum that would be obtained with the main sensor if only the additional parameters had changed between a date t' prior to the analysis date t and the analysis date t. The difference between this expected main sensor datum and the real main sensor datum contains the analyte-specific part of the signal sensed by the main sensor, that is to say the part of the signal that is not related to the additional parameters.

The principal model can then focus on the analyte-specific part of the signal in order to estimate the desired quantity.

The use of two successive models enables to reduce the complexity of the problem to be solved and as a consequence, the quantity to be estimated can be estimated with a better accuracy and with a lower time and memory complexity.

In a particular embodiment of the process for estimating a quantity in mathematical relationship with an analyte concentration level in a target with an analysis, the quantity in mathematical relationship with an analyte concentration level at an analysis date t in a target is chosen in the list {analyte concentration level at the analysis date t, variation of the analyte concentration level between a date t' prior to the analysis date t and the analysis date t, rate of change of the analyte concentration level at the analysis date t, a range of analyte concentration levels}.

Depending on the training of the principal model, it is possible to estimate a variety of quantities in mathematical relationship with the analyte concentration level. For example, if the analyte concentration level is blood glucose, different quantities related with blood glucose (such as blood glucose itself, or the change of the blood glucose between a date prior to the analyses date and the analysis date, or the rate of change of blood glucose at the analysis date) may help the diabetic patient understand his physiological condition or adapt his insulin therapy.

Training the principal model in order to estimate directly the desired quantity rather the analyte concentration level (where appropriate) reduces the complexity of the algorithm.

It is also possible to train the principal model in order to categorize the analyte concentration level to be estimated in a range of analyte concentration levels, for example above or under one or more glycemia thresholds.

In a particular embodiment of the process for estimating a quantity in mathematical relationship with an analyte concentration level in a target with an analysis apparatus, the environmental model further receives as an input at least one additional sensor datum at the prior date t' earlier than the analysis date t and the principal model further receives as input an analyte concentration level at the prior date t'.

In this case, the environmental model can take into account the changes in the additional sensor datum. This enables a more accurate estimation of the expected main sensor data, since the signal sensed by the main sensor varies in general continuously and moderately between two relatively close dates.

In this case, the principal model can estimate the quantity in mathematical relationship with the analyte concentration level in the target based on a preceding analyte concentration level. The principal model can thus work in terms of variations rather than in the absolute. Since the analyte concentration level varies in general slowly and moderately in a human or animal target, the estimation of the principal model can as a consequence be more accurate.

In a particular embodiment of the process for estimating a quantity in mathematical relationship with an analyte concentration level in a target with an analysis apparatus, the wave emitter is a light emitter and the wave generated in the target in response to an irradiation of the target with the light emitter is a thermal wave.

In this case, the process can be implemented on an analyte monitor dealing with an analyte that absorbs at least part of the light emitted by the light emitter.

In a particular embodiment, the process for estimating a quantity in mathematical relationship with an analyte concentration level in a target with an analysis apparatus a further comprises :
- providing a user interface receiving data from one of said at least one processor;
- displaying said quantity in mathematical relationship with the analyte concentration level at the analysis date t on the user interface at a display date t+d where d ranges in [0 min ; 30 min].

In this case, it is possible to proceed to a double check in order to ensure that the quantity displayed is physiologically consistent. For example, in the case where blood glucose is monitored, blood glucose can't vary drastically. If a value of blood glucose estimated at date t, it is possible to check during the delay d if this value is consistent with one or more previous values and if so, to display the estimated value only at the date t+d. Otherwise, a new measurement can be made.

This increases the security of a glucose monitor without significant impact on the insulin therapy if the delay d is moderated, for example less than 30 minutes.

In a particular embodiment of the process for estimating a quantity in mathematical relationship with an analyte concentration level in a target with an analysis apparatus, the irradiation step c, the calculation step d and the calculation step e are iteratively repeated.

In this case, the results of a previous calculation step e can be taken in account in a new calculation step e, for example to calculate mean values in order to improve the accuracy of the estimation.

In this case, it is also possible to continuously monitor a quantity in in mathematical relationship with an analyte concentration level in a target. For example, the process can be implemented on a continuous glucose monitor.

**In** a particular embodiment, the process for estimating a quantity in mathematical relationship with an analyte concentration level in a target with an analysis apparatus further comprises :
f. calculating with a processor a quantity in mathematical relationship with an analyte concentration level at the analysis date t based on at least two quantities in mathematical relationship with the analyte concentration levels at at least two different analysis dates t1 and t2 calculated at two different calculation steps e, said at least two different analysis dates t1 and t2 being in mathematical relation with the analysis date t.

**In** this case, it is possible the estimated quantity in mathematical relationship with an analyte concentration provided for example to a user or a practitioner may be physiologically consistent over time. Erratic values may be suppressed or the noise can be smoothed.

**In** a particular embodiment, the process for estimating a quantity in mathematical relationship with an analyte concentration level in a target with an analysis apparatus comprises prior to the provision of a set of models at step a :
- obtaining a first training dataset with a main sensor and with at least one additional sensor, the first training dataset comprising at least one series of main sensor data and simultaneous additional sensor data, all the main and additional sensor data of a given series being generated in one or more targets having the same analyte concentration level,
- training a first algorithm in a processor with the first training dataset to estimate said environmental model, so that a processor implementing the environmental model can estimate at least one expected main sensor datum at an analysis date t when receiving as input at least one additional sensor datum at said analysis date t and at least one real main sensor datum at a prior date t' earlier than said analysis date t, and
- obtaining a second training dataset with a main sensor, at least one additional sensor and a training sensor configured to invasively sense said analyte concentration level, the second training dataset comprising at least two series of data, each series comprising at at least two given dates at least one real main sensor datum at each given date, the associated at least one expected main sensor datum at said given date, beforehand calculated with a processor implementing the environmental model, and the quantities in mathematical relationship with the analyte concentration level sensed at said given date with the training sensor, any two given series corresponding to two different analyte concentration levels,
- training a second algorithm in a processor with the second training dataset to estimate said principal model so that a processor implementing the principal model can estimate a quantity in mathematical relationship with an analyte concentration level at an analysis date t when receiving as input said at least one expected main sensor datum at an analysis date t and at least one real main sensor datum at this analysis date t.

In a particular embodiment of the process for estimating a quantity in mathematical relationship with an analyte concentration level in a target with an analysis apparatus, the training of any of the first and second algorithms comprises a supervised machine learning technique.

In a particular embodiment of the process for estimating a quantity in mathematical relationship with an analyte concentration level in a target with an analysis apparatus, the target is a human being and any of the first and second datasets of the estimation step comprises data typical of a particular group of human beings to which the target belongs.

This allows to estimate an environmental model and/or a principal model that reflects the physiological specificities of a particular group of human beings and thus to estimate the desired quantity with greater accuracy in case the target can be associated with this particular group.

In a particular embodiment of the process for estimating a quantity in mathematical relationship with an analyte concentration level in a target with an analysis apparatus, the target is a human being and any of the first and second datasets of the estimation step comprises data acquired on the target on which the analyzing apparatus is implemented.

In this case, it is possible to train the environmental model and/or the principal model so that it is specifically adapted to the target on which it is implemented and thus to estimate the desired quantity with greater accuracy.

In a particular embodiment of the process for estimating a quantity in mathematical relationship with an analyte concentration level in a target with an analysis apparatus, the one or more additional parameters comprise physiological parameters chosen in the list {a temperature of the target, a pressure of the target, a pH of the target, a blood volume parameter, a heart rate, a second analyte concentration level in the target}.

These physiological parameters reflect the physiological condition of a human being or of an animal and they generally influence the wave generated in the target as well as the analyte of interest, and they influence as a consequence the signal sensed by a main sensor. Taking account of one or more of these physiological parameters enables to focus on the analyte-specific part of this signal, and thus to estimate the desired quantity with greater accuracy.

In a particular embodiment of the process for estimating a quantity in mathematical relationship with an analyte concentration level in a target with an analysis apparatus, the one or more additional parameters comprise environmental parameters chosen in the list {a temperature of the environment of the target, a pressure in the environment of the target, a humidity level in the environment of the target}.

These environmental parameters generally influence the signal sensed by a main sensor. Taking account of one or more of these physiological parameters enables to focus on the analyte-specific part of this signal, and thus to estimate the desired quantity with greater accuracy.

The invention also relates to an analyte monitor for non invasively estimating a quantity in mathematical relationship with an analyte concentration level in a target comprising
- a wave emitter configured to irradiate the target so that a wave is generated in the target in response to this irradiation,
- a main sensor configured to detect directly or indirectly a wave being generated in a target in response to an irradiation of the target,
- at least one additional sensor configured to sense one additional parameter influencing a signal sensed by the main sensor,
- at least one data processor module configured to receive at least one main sensor datum and at least one or more additional sensors datum and comprising a processor implementing an environmental model receiving as input at least one additional datum at an analysis date t from the one or more additional sensor and at least one real main sensor datum from the main sensor at a prior date t' earlier than said analysis date t, and providing at least one expected main sensor datum at the analysis date t that estimates at least one datum which would have been obtained with the main sensor at the analysis date t if the analyte concentration level had remained the same at the analysis date t as at the prior date t', and a processor implementing a principal model receiving said at least one expected main sensor datum at the analysis date t from said environmental model and at least one real main sensor datum from the main sensor at the analysis date t, and estimating a quantity in mathematical relationship with an analyte concentration level at the analysis date t.

**In** a particular embodiment of the analyte monitor, the wave emitter is a light emitter and in that the wave generated in the target in response to an irradiation of the target with the light emitter is a thermal wave.

**In** this case, it is possible to choose a light that is at least partially absorbed by the analyte of interest and obtain information on the analyte concentration in the target by sensing the thermal wave directly or indirectly.

**In** a particular embodiment of the analyte monitor, the at least one additional sensor is chosen in the list {temperature sensor, pressure sensor, pH-sensor, moisture sensor, humidity sensor, photoplethysmograph, analyte concentration sensor}.

**In** a particular embodiment of the analyte monitor, the analyte the concentration level with which the quantity to be estimated is in mathematical relationship is blood glucose and in that the analyte monitor is a continuous monitor.

**In** this case, the analyte monitor can for example be used to provide data to an insulin therapy delivery system.

The disclosure also relates to a processor implementing an environmental model receiving as input
- at least one additional datum at an analysis date t at which a quantity in mathematical relationship with an analyte concentration level in a target is to be estimated from at least one additional sensor configured to measure at least one additional parameter influencing a signal sensed by a main sensor configured to sense directly or indirectly a wave being generated in the target in response to an irradiation of the target with a wave emitter,
- and at least one real main sensor datum from the main sensor at a prior date t' earlier than said analysis date t,
and providing at least one expected main sensor datum at the analysis date t that estimates at least one datum which would have been obtained with the main sensor at the analysis date t if the analyte concentration level in the target had remained the same at the analysis date t as at the prior date t'.

The disclosure further relates to a processor implementing a principal model receiving at least one expected main sensor datum at an analysis date t from a processor implementing an environmental model and at least one real main sensor datum at the analysis date t from a main sensor configured to sense directly or indirectly a wave being generated in a target in response to an irradiation of the target with a wave emitter, and estimating a quantity in mathematical relationship with an analyte concentration level in the target at the analysis date t.

The invention relates in addition to a computer program comprising instructions which, when the program is executed by a computer, cause the computer to carry out the process for estimating a quantity in mathematical relationship with an analyte concentration level in a target with an analysis apparatus according to any of the embodiment described above.

### BRIEF DESCRIPTION OF THE DRAWINGS

Embodiments of the invention will be described below, in relation to the following drawings :
[Fig. 1] shows a schematic representation of an analyte monitor in accordance with exemplary embodiments.
[Fig. 2] shows a classical workflow for measuring an analyte value with an analyte monitor according to figure 1.
[Fig. 3] shows a workflow according to one embodiment of the invention for measuring an analyte value with an analyte monitor according to figure 1.
[Fig. 4a] shows the variations over time of a first additional parameter sensed with a first additional sensor.
[Fig. 4b] shows the variations over time of a second additional parameter sensed with a second additional sensor.
[Fig. 4c] shows the output over time of an environmental model at date t receiving as input data from the first and second additional sensors of figures 4a and 4b overlaid with the measurements of the main sensor.
[Fig. 5] shows the estimation of the glycemia AV(t)_inventionobtained with the analysis apparatus in an extremely simplified case, in which the analysis apparatus comprises a photoacoustic detection cell to monitor blood glucose and one assumes that the photoacoustic signal depends only on blood glucose and the external temperature compared to the glycemia reference value AV(t).
[Fig. 6] shows an exemplary workflow for estimating an environmental model.
[Fig. 7] shows a first exemplary workflow for forming a second training dataset.
[Fig. 8] shows an exemplary workflow for estimating a principal model based on the second training dataset of figure 7.
[Fig. 9] shows a second exemplary workflow for forming a second training dataset.

On the drawings, the same reference signs show the same or similar objects.

### DETAILED DESCRIPTION

The invention relates to a process for estimating a quantity in mathematical relationship with an analyte concentration level in a target, with an non invasive analysis apparatus 1, and also to an analysis apparatus 1 or an analyte monitor 1 for non invasively estimating a quantity in mathematical relationship with an analyte concentration level.

The analysis apparatus 1 may be used to analyze a target 2 and more specifically to measure a substance or analyte in a target 2. The target 2 is for example a human or an animal tissue, like skin.

As a non limiting example, the analyte to be measured is blood glucose. More specifically, a blood glucose value or a quantity in mathematical relationship with blood glucose can be estimated based on a non-invasive interstitial glucose measurement.

This analyte will be called "principal analyte" in the following description.

In one embodiment, the analysis apparatus 1 can be portable or wearable.

The analysis apparatus 1 for non invasively estimating a quantity in mathematical relationship with an analyte concentration level in a target comprises :
- at least one wave emitter configured to irradiate the target so that a wave, called generated wave, is generated in the target in response to this irradiation,
- a main sensor configured to detect directly or indirectly a wave being generated in a target in response to an irradiation of the target,
- at least one additional sensor configured to sense one additional parameter influencing the signal sensed by the main sensor,
- at least one data processor module configured to receive at least one main sensor datum from the main sensor and at least one additional datum from the at least one additional sensor and comprising :
   a) a processor implementing an environmental model receiving as input at least one additional datum at an analysis date t from the at least additional sensor and at least one real main sensor datum from the main sensor at the prior date t' earlier than said analysis date t, and providing at least one expected main sensor datum at the analysis date t that estimates at least one datum which would have been obtained with the main sensor at the analysis date t if the analyte concentration level had remained the same at the analysis date t as at the prior date t',
   b) and a processor implementing a principal model receiving said at least one expected main sensor datum at the analysis date t from said environmental model and at least one real main sensor datum from the main sensor at the analysis date t, and estimating a quantity in mathematical relationship with an analyte concentration level at the analysis date t.

In a particular embodiment, the wave emitter, the main sensor, the one or more additional sensors and the processor module are enclosed in the same housing.

In a particular embodiment, the environmental model and the principal model are implemented on the same processor.

The wave emitter can for example be a light emitter or emit an acoustic wave.

The wave emitted towards the target 2, called irradiation wave, interacts with the target so that another wave, called generated wave is generated in the target 2 in response to the irradiation wave.

The generated wave depends on the irradiation wave but also on the characteristics of the target 2. If the generated wave propagates outside the target 2, it also depends on the environment of the target 2.

The nature and/or the Fourier spectrum, in particular the amplitude or the phase of a component of the generated wave, can depend on the target 2 and/or on its environment.

For example, the generated wave may be the consequence of an absorption phenomenon or/and of a reflection phenomenon and/or of a scattering phenomenon in the target 2. As a consequence, the generated wave contains information about the composition and/or the structure of the target 2. If the irradiation wave is appropriately chosen, the generated wave can thus be exploited to gain insight on an analyte concentration level in the target 2.

In an exemplary embodiment, the wave emitter is a light emitter, emitting an electromagnetic wave towards the target. In this case, the generated wave can be a thermal wave.

In this case, the analysis apparatus 1 or analyte monitor 1 may comprise:
- a light emitter emitting an intensity-modulated light, an intensity-modulation device, a light emitter controller controlling at least a modulation frequency at which said intensity-modulation device modulates the intensity of a light emitted by the light emitter,
- at least one detection cell 12 comprising a sensor, called "main sensor", sensing directly or indirectly a thermal wave propagating out of a target 2 in response to an irradiation of the target by the emitted light,
- at least one additional sensor 14, called "complementary sensor" or "additional sensor",
- and a processor module 13 configured to receive sensor data from the main sensor and the one or more additional sensor and process these data.

The analysis apparatus 1 may be based on the photoacoustic detection. In this case, the detection cell 12 is a photoacoustic (PA) detection cell.

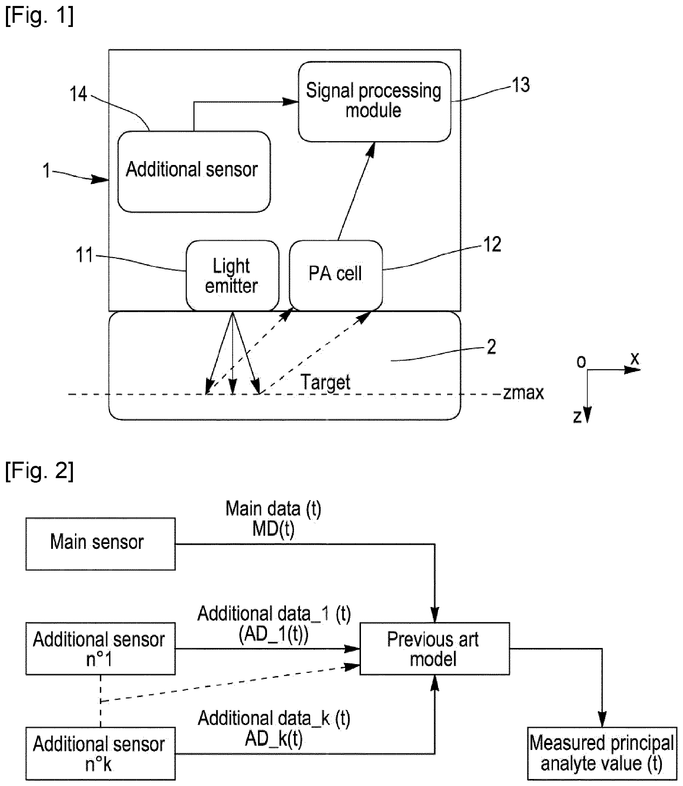

Figure 1 shows a schematic diagram illustrating how photoacoustic measurement techniques may be used to non-invasively obtain an analyte sensor signal representative of an analyte concentration, for example a blood glucose concentration level of a user.

As can be seen on figure 1, a light emitter block 11 may comprise a light emitter configured to emit a light beam towards a target 2. The target 2 could be a patient's tissue, for example skin.

The light incident on the target 2 penetrates a distance into the target 2 and interacts with analyte molecules that are present inside the target 2, for example glucose molecules.

The analyte molecules are in consequence thermally excited.

As a result, a thermal wave propagates in the target 2, in particular towards the surface of the target 2 and, then into the medium surrounding the target 2, for example in the air.

In case a gaseous medium is surrounding the target 2, an acoustic wave associated with the thermal wave propagates in this gaseous medium surrounding the target 2

Consequently, the thermal wave propagating in response to the irradiation of the target 2 can be directly or indirectly sensed by the main sensor.

In a first case, the thermal wave can be directly sensed with a thermal sensor which is comprised in the detection cell 12. That is to say, in case the thermal wave is directly sensed, the main sensor is a thermal sensor configured to sense the thermal wave.

In a second case, the main sensor can be a photoacoustic sensor configured to detect the acoustic wave associated with the thermal wave in a gaseous medium surrounding the target 2.

The propagation of the light emitted by the light emitter (or equivalently the irradiation wave) and of the thermal wave is schematically illustrated in figure 1 through the use of respectively bold and dashed arrows.

In case the thermal wave is indirectly sensed by a photoacoustic detection cell 12, this detection cell comprises a chamber filled with a gas (for example air) through which the acoustic wave propagates, and one or more appropriate sensors placed in this chamber, for example opposite the target 2.

In an exemplary embodiment, the main sensor is an electroacoustic sensor configured to convert the pressure of the acoustic wave into an electrical signal, such as a microphone. In an alternative exemplary embodiment, the photoacoustic detection cell 12 comprises a transducer, for example a piezoelectric transducer.

In exemplary embodiments, the main sensor is operably connected to a signal processing module 13. The signal processing module 13 comprises an analog-to-digital converter configured to convert the analog electrical signal from the electroacoustic sensor to a digital signal.

The signal processing module 13 optionally comprises an operational amplifier operably connected to the analog-to-digital converter and configured to further amplify an electronic signal derived from the acoustic response of the target 2.

In exemplary embodiments, the analog-to-digital converter is operably connected to a digital signal processor for processing of the digital signal.

In a particular embodiment, the light emitter emits an intensity-modulated laser beam at at least one particular wavelength towards the target 2.

In an exemplary embodiment, the light emitter is a light-emitting diode (LED).

In an alternative embodiment, the light emitter is a laser chip.

For example, the light emitter comprises a Quantum Cascade Laser (QCL) emitting in the mid-infrared region (MIR-QCL).

The light emitter block 11 can also comprises the circuitry associated with the light emitter and a controller module configured to control the light emitter such that the intensity of the light emitted by the light emitter is modulated at a tunable intensity modulation frequency.

The intensity modulation can be obtained by any known electric or mechanic means.

The light can be emitted continuously or in a pulsed manner.

In a particular embodiment, the light emitter controller module is configured to constrain the light emitter so as to emit light pulses. The pulses may have durations of about 100 nanoseconds (ns) to 500 ns per pulse, and a duty cycle for example of 1% to 20%, which corresponds to a frequency of the order of 20 kHz to 2 MHz.

It must be understood that, in order to help understand the invention, a blood glucose sensor will often be described in the detailed examples below, but another analyte can be measured in the same way by merely adapting the wavenumber of the light emitted by the light emitter to the analyte to be monitored.

The process and system described here can also be adapted to other targets or tissues than skin. For example, biological fluids such as saliva could be analyzed.

In an exemplary embodiment, at least one wavelength λ_{irrad} of the light emitted by the light emitter is selected so that the light strongly interacts with an analyte of interest.

In case an analyte of interest is glucose, the wavelength of the light emitted by the light emitter can be selected to correspond to wavelengths that interact strongly with glucose molecules for thermal excitation of the glucose molecules.

For example, lights having wavenumbers (the reciprocal of wavelength) of between about 1150 cm⁻¹ and 1000 cm⁻¹ interact strongly with glucose molecules. This wavenumber range corresponds to wavelengths in the mid-infrared (MIR) region.

Preferably, but not mandatorily, the analysis apparatus directly contacts the target 2. For example, at least part of the detection chamber of a photoacoustic detection cell 12 is in direct contact with skin.

In addition to the main sensor, the analysis apparatus 1 comprises at least one additional (or complementary) sensor 14, configured to detect a signal correlated with a parameter influencing the signal sensed by the main sensor, the parameter being different from the principal analyte concentration to be measured.

If the generated wave is a thermal wave, the parameter can for example directly influence the thermal wave, or it can influence the acoustic wave in case the thermal wave is indirectly sensed with a photoacoustic sensor.

In other words, an additional sensor 14 is configured to measure at least one additional parameter influencing directly or indirectly the physical phenomenon sensed by the main sensor.

Such a parameter will be called "complementary parameter" or "additional parameter" in the following description.

Two types of additional parameters can be taken into account.

First, an additional parameter may be a so-called "environmental parameter", that is to say a physical or chemical parameter related to the environment of the target 2.

Indeed, whatever the type of the main sensor, the generated wave propagating outside the target 2 and detected by the main sensor depends on the environment of the target 2.

For example, the additional sensor can measure a temperature such as the temperature in the air in the detection cell 12 or surrounding the detection cell 12. It can also measure the pressure of the air in the detection cell 12 or surrounding the detection cell 12, or the humidity level in or in the vicinity of the detection cell 12.

Such environmental parameters influence in particular the speed of sound and influence as a consequence the signal sensed by the main sensor.

An additional parameter can also be a so-called "physiological parameter", that is to say a parameter related to the target 2 itself.

A physiological parameter can for example be the water content of the tissue to be analyzed, the concentration of another molecule (called "secondary analyte") than the principal analyte, blood volumetric changes, etc.

Hence, as a non-limiting list, an additional sensor can be a thermal sensor, a pressure sensor, a humidity sensor, a photoacoustic sensor operating at at least one other wavelength and/or intensity modulation frequency than the main sensor (where applicable), a photoplethysmography (PPG) sensor, a pH sensor, a moisture sensor, a heart rate sensor, a microphone or an impedance sensor.

One or more additional sensors can be implemented simultaneously. The choice of the type(s) and/or the number of additional sensors may result from a compromise between the accuracy of the analysis and the wearability and / or the power consumption of the analysis apparatus 1.

Depending on its type, a complementary sensor may be positioned inside or outside the detection cell 12 where applicable, and in direct contact with the target 2 to be analyzed or not.

As seen above, numerous environmental parameters can influence the wave (or more generally the physical phenomenon or signal sensed by the main sensor) generated in response to the irradiation of the target 2 with the wave emitter, in addition to the principal analyte concentration. As a consequence, deriving a principal analyte concentration or a quantity in mathematical relationship with a principal analyte concentration from the measured signal is extremely complex.

To address this problem, it has already been proposed to use additional sensors in addition to a main sensor. However, up to date, the data from all the sensors (main and additional) are systematically analyzed as a whole, as can be seen on the classical workflow represented on figure 2, which does not reduce the complexity of the problem.

Further, since the amplitude of the changes in the thermal wave (or more generally the physical phenomenon sensed by the main sensor) due to the changes in the principal analyte concentration and the characteristic time of these changes may be very different from those due to one or more additional parameters, one cannot guarantee that the principal analyte concentration (or the quantity in mathematical relationship with this concentration) is accurately measured with this classical workflow or that the additional sensor data helps increase the accuracy of the measurement of the analyte concentration.

In a classical workflow, the data from all the sensors at an analysis date t constitute the input of a single model, the output of which is an estimated concentration of the principal analyte at the analysis date t.

The method according to the invention relies on the assumption that the respective contributions of the principal analyte concentration and of the environmental parameters to the signal sensed by the main sensor or to the changes in the signal sensed by the main sensor can be dissociated from each other, in order to be able to focus on the principal analyte-specific part of the signal sensed by the main sensor.

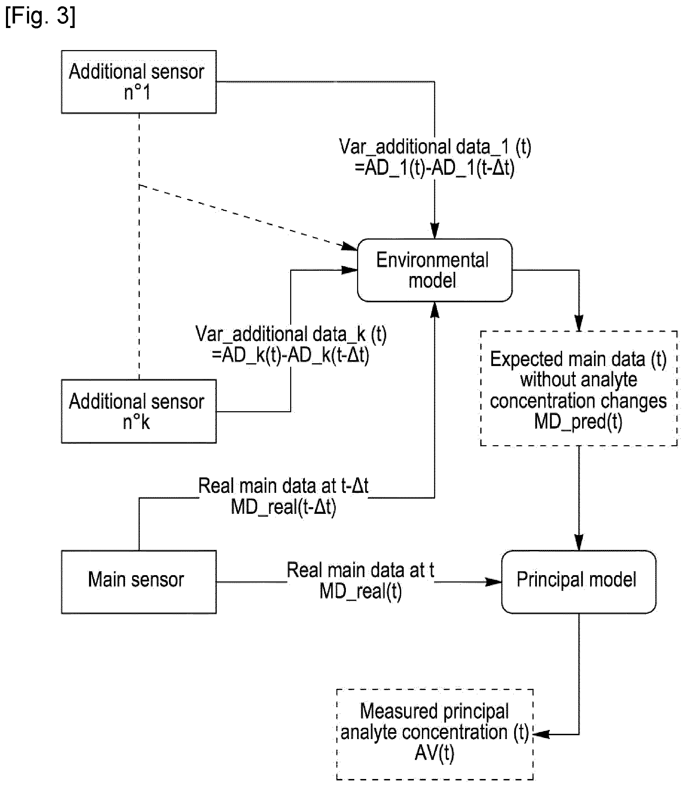

To this purpose, the process according to the invention comprises:
- providing at least two different models, respectively called "environmental model" and "principal model", as represented on figure 3;
- triggering the irradiation of a target 2 with a wave emitter at a date t' earlier than an analysis date t, called "prior date t'", in order to generate a first wave in the target 2, directly or indirectly detecting this first generated wave with the main sensor and receiving at least one real main sensor datum MD_real(t') from the main sensor at the prior date t',
- triggering the irradiation of the target 2 with the wave emitter at the analysis date t in order to generate a second wave in the target 2, directly or indirectly detecting this second generated wave with the main sensor, measuring at least one additional parameter with at least one additional sensor and receiving and at least one additional datum AD_i(t) at the analysis date t from at least one additional sensor and at least one real main sensor datum MD_real(t) at the analysis date t from the main sensor,
- calculating at least one expected main sensor datum MD_pred(t) at the analysis date t based on the environmental model, on the at least one additional datum AD_i(t) at the analysis date t and on the at least one real main sensor datum MD_real(t') at the prior date t' with a processor implementing the environmental model,
- calculating with a processor implementing the principal model a quantity in mathematical relationship with an analyte concentration level at the analysis date t based on the principal model, the at least one real main sensor datum MD_real(t) at the analysis date t and the at least one expected main sensor datum MD_pred(t) at the analysis date t calculated with the processor implementing the environmental model.

The inputs of the first model, also called "environmental model", comprise at least one additional sensor datum at the analysis date t. If k additional sensors are implemented, the environmental model receives at least one additional sensor datum AD_1(t) from a first additional sensor at an analysis date t, at least one additional sensor datum AD_2(t) from a second additional sensor at the same analysis date t , etc., up to at least one additional sensor datum AD_k(t) received form the k-th additional sensor at the same analysis date t.

At the same time, the inputs of the environmental model also comprise at least one main sensor data at another date (date t-Δt or prior date t' earlier than the analysis date t), also called MD_real(t-Δt) where Δt is a positive predetermined duration.

Based on these inputs, the environmental model provides as an output at least one predicted main sensor datum at the analysis date t (also called expected main sensor datum at date t, MD_pred(t)).

The main sensor data acquired by the main sensor are said "real" data in that they are obtained based on the sensing of a physical phenomenon. They are as real as the additional sensor data. The term real is added in the case of the main sensor data in order to facilitate the understanding and to distinguish the "real" main sensor data from the "expected" main sensor data, which are not acquired by the main sensor but rather calculated with a processor implementing the environmental model.

Since the at least one main sensor predicted value or datum is based on at least one real main sensor datum at a prior date t-Δt,on at least one additional sensor datum at the analysis date t and on the environmental model trained with series of data with a constant principal analyte level as described below, one can consider that this predicted main sensor datum corresponds to the main sensor datum that the main sensor would have detected at the analysis date t if only the additional parameters had been allowed to change in the way they did between the two dates t-Δt and t, and not the principal analyte concentration.

In other words, a main sensor predicted (or expected) datum takes into account the real changes of one or more additional parameters with a constant principal analyte concentration.

In a particular embodiment, the environmental model also receives at least one additional sensor datum at the prior date t-Δt. For example, the first model may also receive as an input at least one additional sensor datum at the prior date t-Δt, or it may directly receive the variation of at least one additional sensor datum between the prior date t-Δt and the analysis date t: AD_i(t) -AD_i(t-Δt), i ranging from 1 to k, rather than the additional sensor datum at the prior date t-Δt and/or at the analysis date t, as represented on figure 3.

In this case, the environmental model is based on the changes of the additional parameters at a time scale corresponding to the predetermined duration Δt. If the predetermined duration Δt is appropriately chosen, the accuracy of the environmental model may be enhanced relatively to an environmental model based only on additional sensor data at the analysis date t.

The output of the environmental model - that is to say the at least one main sensor predicted datum at an analysis date t (MD_pred(t)) - is the first input of the second model, called "principal model".

The principal model also receives as an input at least one real main sensor datum at an analysis date t (MD _real(t)).

The output of the principal model is an estimation of a quantity in mathematical relationship with the principal analyte concentration at the analysis date t, or equivalently, a measurement of a quantity in mathematical relationship with the principal analyte concentration at the analysis date t.

For example, the output of the principal model can be an estimation of the principal analyte concentration level at the analysis date t (AV(t)), as shown on figure 3.

In another embodiment, the output of the principal model can be an estimation of the variation of the principal analyte concentration level between the prior date t-Δt and the analysis date t (AV(t)- AV(t-Δt)).

Since the principal model receives as an input at least one expected main sensor data value at an analysis date t and the at least one main sensor predicted datum at the analysis date t, one understands that this model can focus on the principal analyte-specific part of the main sensor data, for example on the basis of a comparison between MD_pred(t) and MD_real(t).

As a consequence, the principal analyte concentration measurement or monitoring with the workflow according to the invention is more accurate and allows a higher sensitivity than with a classical workflow.

According to various embodiments, one may use various numbers, types and positions of additional sensors.

According to various embodiments, different values can be set for the predetermined duration Δt.

For example, in case the method is implemented on a continuous glucose monitoring system, the predetermined duration Δt may be of the order of 10 minutes, 5 minutes or less.

In one embodiment, the measurement is repeated at least two times or iteratively.

In this case, the analysis apparatus 1 can be a continuous analyte monitor apparatus, such as a continuous glucose monitor. Such a continuous glucose monitor can transmit data to an insulin delivery system such as an insulin pump.

For example, at least two quantities in mathematical relationship with the principal analyte level are estimated at two different analysis date t1 and t2 in mathematical relationship with a third analysis date t3.

In this case, the quantity in mathematical relationship with an analyte concentration level at the third analysis date t3 can be based on the at least two quantities in mathematical relationship with the analyte concentration levels at the at least two different analysis dates t1 and t2. For example, it can be an average of these at least two quantities in mathematical relationship with the analyte concentration levels at the at least two different analysis dates t1 and t2.

In a particular embodiment, a user interface may be provided and receive data from the analyte monitor 1 so that the quantity in mathematical relationship with an analyte concentration level at the analysis date t can be transmitted and displayed on a user interface with a delay.

The quantity in mathematical relationship with an analyte concentration level at the analysis date t can for example be displayed on the user interface at a display date t+d where d ranges in [0 min ; 30 min].

In one embodiment, the process according to the invention comprises of the training an algorithm of the signal processor 13 for estimating an environmental model.

The process according to the invention can comprise obtaining a first training dataset based on a first raw dataset for the estimation of the environmental model.

To this purpose, one or more measurement campaigns are carried out, in vivo and/or in vitro and/or in silico.

The in vivo measurement campaigns can be carried out with or one or more animals and/or human beings. They can in particular be adapted to form a first training dataset for an environmental model involving at least physiological additional parameters.

In silico measurement campaigns may be based on theoretical models or equations and may be sufficient for obtaining a first training dataset for an environmental model involving environmental additional parameters.

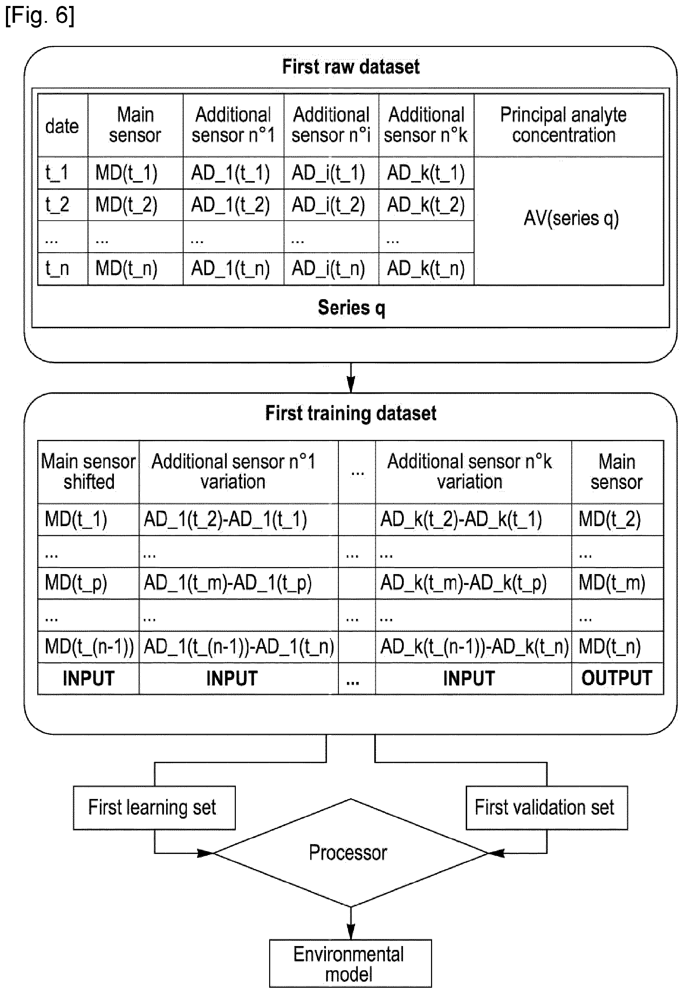

A first raw dataset allowing to form a first training dataset may comprise at least one series of data tuples, each tuple comprising all the additional sensor data sensed at a given date t_i and the real main sensor data MD_real(t_i) at the given date t_i, as shown on figure 6.

All the data tuples of a given series of the first raw dataset correspond to the same analyte concentration value : AV(series q)=AV(t_i), whatever the date t_i. In other words, one tuple of a given series of the first raw dataset, called series q, can be represented as : (MD _real(t_i) ,AD_1(t_ i), AD_2(t _i),...,AD_k(t_i), and optionally : AV(series q)).

A second sensor may be provided in order to make sure that the analyte concentration value is indeed constant for all the tuples of a series : AV(series q)=AV(t_i) whatever the date t_i. The second sensor can be an invasive analyte sensor or a non-invasive analyte sensor, based on another principle than the main sensor.

It is also possible to carry out the measurements without this second sensor, provided that the experimental conditions are chosen so that the analyte concentration value remains constant at the desired precision.

For example, in the case of blood glucose, a measurement campaign may be carried out on a non diabetic patient or a diabetic patient having a stable glycaemia during one or more time intervals. For example, a measurement campaign may be carried out one hour or more away from a meal, or at night.

Based on a first raw dataset, one can form a first training dataset comprising at least one training series of training data tuples. Each training data tuple of a given training series comprises two different real main sensor data MD(t_m) and MD(t_p) at two different dates t_m and t_p, and the variations of part of or all the k additional sensor data between the dates t_m and t_p : AD _i(t__p) -AD_i(t_m), i ranging from 1 to k.

The main sensor data at a first date t_m (MD(t_m)) and the variation of the additional sensors data between the first date t_m and a second date t_p ( AD_i(t__p) - AD -i(t- m) ( i ranging from 1 to k)) are the inputs of the environmental model at the training step, the known main sensor data at the second date t_p (MD(t_p)) being the output to be predicted by the environmental model at the training step.

If a given series of a first raw dataset comprises n data tuples, the corresponding training series of a first training dataset comprises up to n(n-1)/2 training tuples.

The first training dataset may be stored on a mass storage device.

As far as possible, the tuples of a training series of the first training dataset are evenly distributed throughout a given volume in the multidimensional space of the additional parameters.

The volume to be considered in this multidimensional space is finite : the range of the skin temperature is typically [20°C-40°C], the range of the external temperature is typically [-10°C, 40°C], the range of relative humidity is [0%, 100%].

The first training dataset can comprise only one series of training tuples. It can also comprise several training series of training tuples, each training series having a different principal analyte value AV(series q), so as to take into account non-linearities in the typical range of the principal analyte concentration.

In one embodiment, the first training dataset is obtained on a given animal or human being.

In another embodiment, the first training dataset comprises data from a plurality of animals or human beings.

In the case of a blood glucose monitor, the first training dataset can comprise only data from one or more normal human beings ("normal" must be understood here with regards to the analyte to be measured. In this case, a normal human being is somebody who doesn't suffer from diabetes). It can also comprise only data from one or more diabetic human beings.

In one embodiment, the first dataset can comprise data from a patient on which the analysis apparatus is to be implemented.

At the environmental model training step, the algorithm used by the signal processor 13 is trained using the first training dataset. Once the environmental model is trained, a processor implementing the environmental model is able to predict an expected main sensor data MD_pred(t) at a given date t based on one or more additional sensor data at the given date t and on the main sensor data at a date t' and, optionally, one or more additional sensors data at date t'.

The algorithm of the environmental model may be trained directly on the signal processor 13 or on another processor.

Since all the tuples of a given series q of the first training dataset have the same principal analyte concentration value AV(series_q), the expected main sensor data at date t (MD_pred(t)) will be related only to the changes of one or more additional parameters (for example estimated with Var_additional data_i (t)=AD _i(t)- AD_i(t- Δt), i ranging from 1 to k), and not to the changes of the principal analyte concentration.

If needed for the environmental model training, the changes of one or more additional parameters and/or the changes in the main sensor data can be deduced from the first raw dataset as explained above and as shown in figure 6. Since a given series of the first raw dataset comprises at least two tuples corresponding to at least two different dates t _i and t_i', differences between additional or main sensor data at these at least two different dates may be calculated.

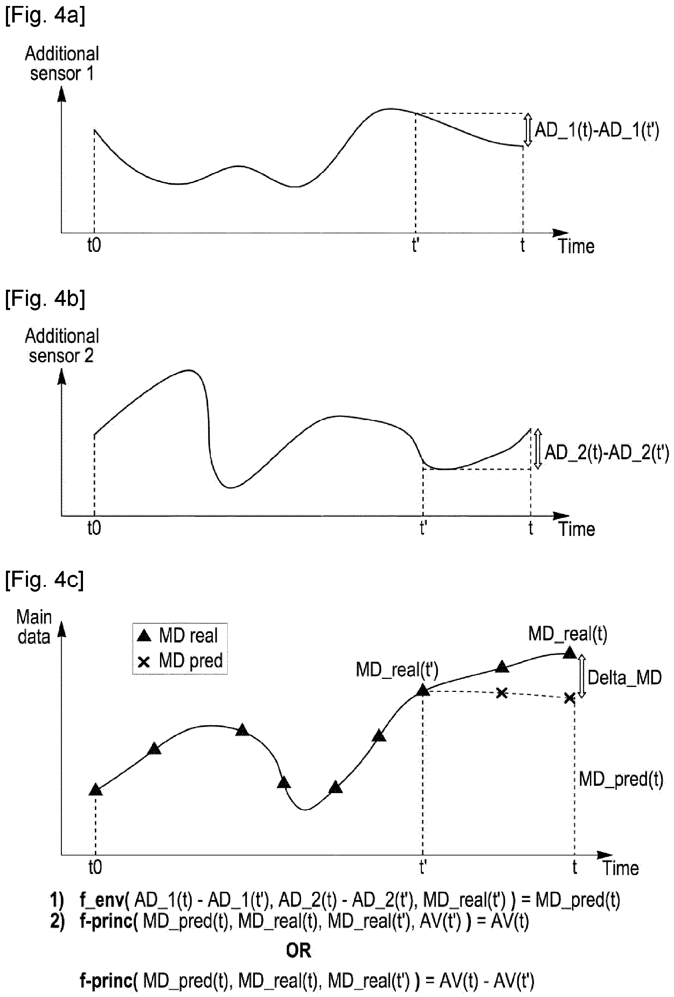

The interest or use of the environmental model is schematically represented in an extremely simplified case on figures 4a, 4b, 4c.

In this case, we assume that the generated wave is a thermal wave. This thermal wave is indirectly sensed with a photoacoustic sensor and that the photoacoustic signal depends only on the external temperature around a human being, measured with the first additional sensor (additional sensor 1), on the moisture around this human being, measured with the second additional sensor (additional sensor 2), and on the blood glucose concentration to be determined.

In this case, an environmental model f_env is trained with a first training dataset comprising at least one first training series with a constant glycaemia but variable temperature and moisture.

At each measurement point, the two additional data are acquired, as represented on figure 4a and figure 4b, as well as the main sensor data, as shown on figure 4c.

In this example, a processor implementing the environmental model (f_env) receives as input the variations of the two additional sensors data between dates t' and t (AD_1(t)-AD_1(t') and AD_2(t)-AD_2(t')) and the main sensor data at the date t' prior to date t (MD _real(t')).

The processor implementing the environmental model calculates based on these inputs a predicted main sensor data at the analysis date t (MD_pred(t)), which may be different from the real main sensor data MD_real(t) at this date t, as represented on figure 4c.

The difference between MD_pred(t) and MD _real(t) isn't due to the changes in the two additional sensors data since these changes are reflected by MD_pred(t). As a consequence, if no other parameter influences the main sensor data than temperature, moisture and glycaemia, as assumed in this case, the difference between MD_pred(t) and MD _real(t) is only correlated to the changes in the glycaemia and can be used to determine either the absolute value of the principal analyte concentration at the analysis date t (AV(t)) - in this case the glycaemia - or the variation of this concentration between date t' and the and the analysis date t (AV(t)-AV(t')).

To this purpose, a principal model f_princ is required.

The estimation of a principal model f_princ will be described below.

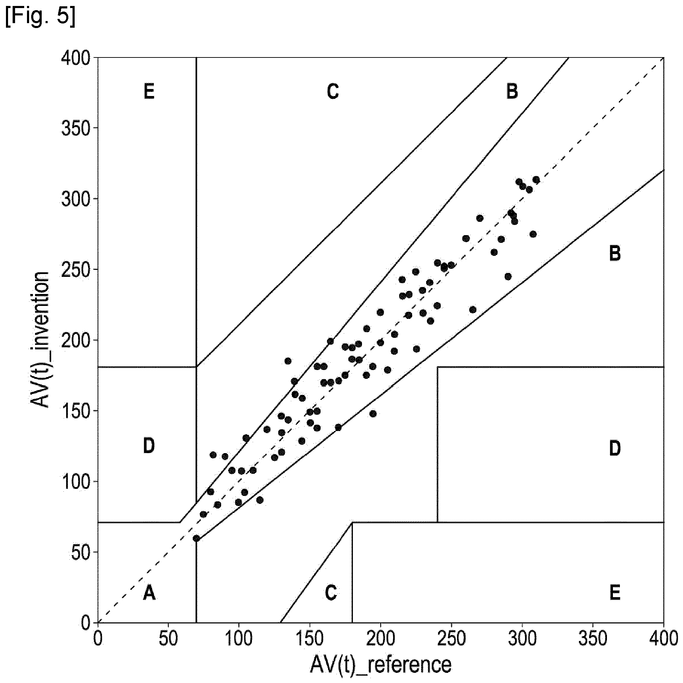

We can already see on figure 5 that, based on the real main sensor data at the analysis date t (MD _real(t)), the real main sensor data at a date t' prior to the analysis date t (MD _real(t')), the predicted main sensor data at the analysis date t (MD_pred(t)) calculated with a processor implementing the environmental model and optionally the principal analyte concentration level estimated or measured at the prior date t' (AV(t')), a processor implementing the principal model is able to estimate the principal analyte concentration level at date t (AV(t) - in the cases of figure 4c and figure 5, the glycaemia).

The environmental model can be based on any supervised machine learning technique, such as a random forest, neural networks, etc.

Optionally, the first training dataset comprises several series, two series corresponding to two different principal analyte concentrations.

The first training dataset may be divided into two different sets : a first learning set and a first validation set. For example, 80% of the first training dataset is dedicated to the learning set and 20% of the first dataset is dedicated to the validation set.

The environmental model can also be based on known theoretical relationships between any of the influencing parameters and the physical phenomenon sensed by the main sensor.

The process according to the invention can comprise the training of an algorithm of the signal processor 13 for estimating a principal model.

The principal model can be trained once the environmental model is trained.

The training of the principal model requires a second training dataset. To form the second training dataset, a second raw dataset is needed.

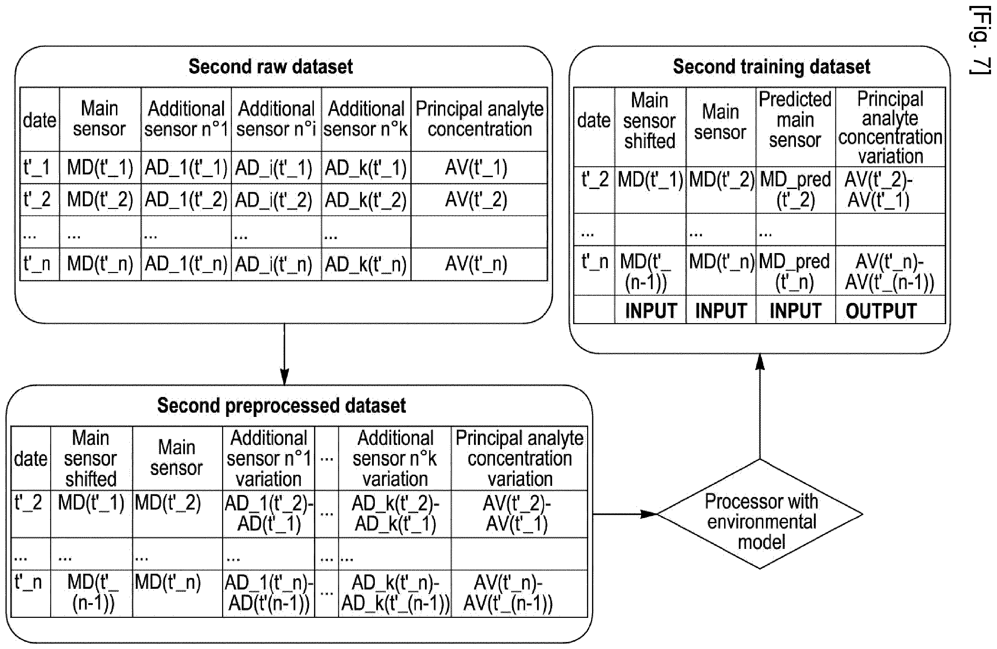

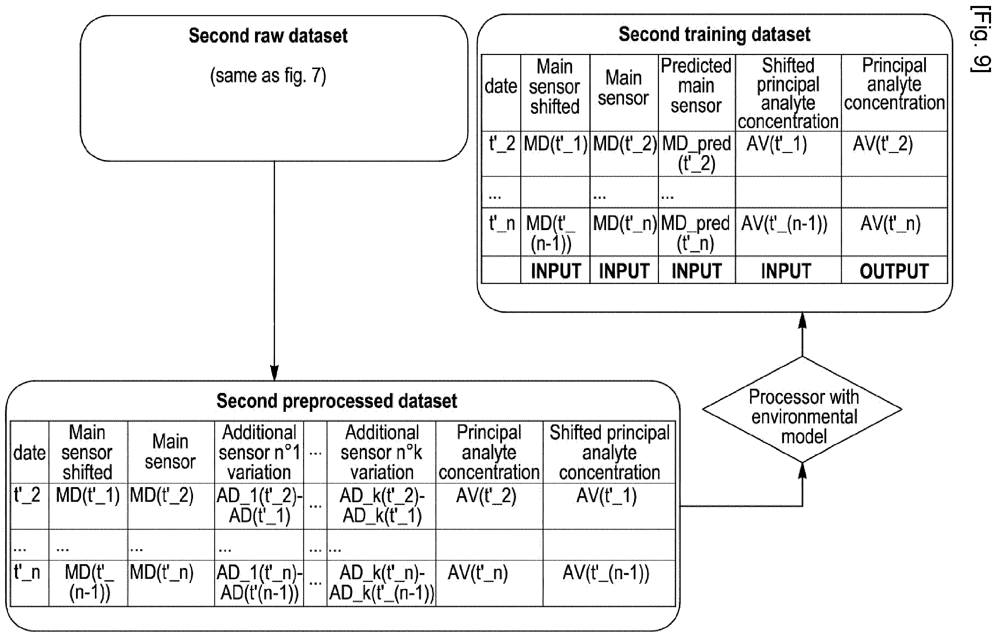

The second raw dataset comprises at least one data tuple, each data tuple comprising all the additional sensor data sensed at a given date t_p and the real main sensor data MD_real(t'_p) at the given date t'_p, as shown in figures 7 and 9.

The first and the second raw datasets may share one or more tuples, but in the case of the second raw dataset, the principal analyte concentration AV (t'_p) may be different between any two tuples.

Based on the second raw dataset, a processor forms a preprocessed dataset, called second preprocessed dataset, each tuple of the second dataset comprising :
- all the input data needed for the environmental model. In the embodiments represented on figures 7 and 9, theses data are the additional sensors variations between a given date t'_p and a previous date t'_(p-1) : AD_i (t'_p)-AD _i(t'_(p-1)), i ranging from 1 to k, and the real main sensor data at the previous date t'_(p-1) (MD _real(t'_(p-1)), written in a simplified manner MD(t'_(p-1)) in figures 7, 8 and 9);
- the real main sensor data at the given date t'_p (MD _real(t'_p)) and at another date t'_(p-1) (MD_real(t'_(p-1)), written in a simplified manner MD(t'_p)) and MD(t'_(p-1)) in figures 7, 8 and 9);
- either the variation of the principal analyte concentration level between the given date t'_p and the previous date t'_(p-1), as represented on figure 7,
- or the principal analyte concentration levels at the given date t'_p and at the previous date t'_(p-1).

A processor implementing the environmental model calculates for each tuple the predicted main sensor data MD_pred(t'_p).

Based on the second preprocessed dataset and the corresponding main sensor data, a second training dataset may be formed.

Each tuple of the second training dataset comprises :
- the predicted main sensor datum or data at a given date t'_p (MD_pred(t'_p)) ;
- the real main sensor data at the the given date t'_p (MD _real(t'_p), written in a simplified manner MD(t'_p) in figures 7, 8 and 9);
- optionally, the real main sensor data at the previous date t'_(p-1) (MD _real(t'_(p-1)), written in a simplified manner MD(t'_(p-1)) in figures 7, 8 and 9);
- either the variation of the principal analyte concentration level between the given date t'_p and the previous date t'_(p-1), as represented on figure 7 to be estimated.
- or the principal analyte concentration levels at the given date t'_p to be estimated and at the previous date t'_(p-1), as represented on figure 9.

In a first embodiment, once the principal model estimated, a processor implementing the principal model receives as input at least one real main sensor datum at a given date t MD_real(t) and the corresponding one or more expected main sensor data MD_pred(t) calculated by a processor implementing the environmental model (which receives as inputs at least one ore more additional sensor datum at date t and on at least one real main sensor datum at a date t-Δt earlier than date t), and optionally at least one real main sensor datum at a previous date t- Δt MD_real (t-Δt).

The processor implementing the principal model returns as an output a quantity in mathematical relationship with the principal analyte concentration level at the given date t.

The quantity in mathematical relationship with the principal analyte concentration level at the given date t may be in a first embodiment the variation of the analyte concentration between the previous date t - Δt and the analysis date t (AV(t)-AV(t - Δt)).

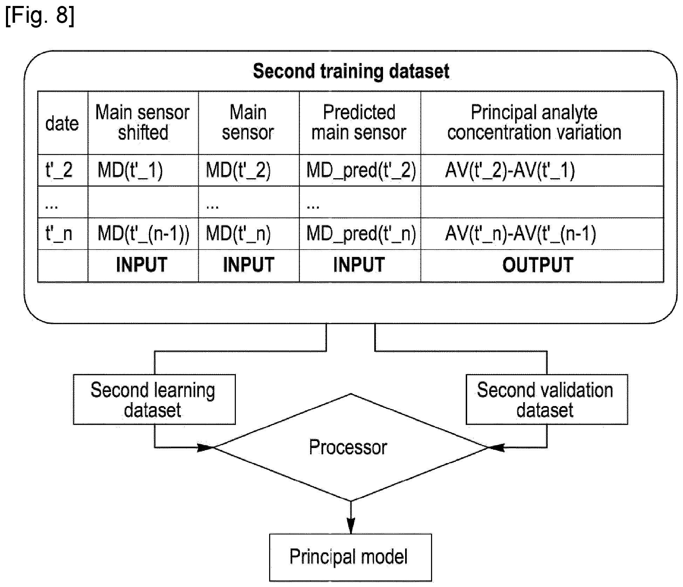

This first embodiment is represented in figures 7 and 8. In this case, no additional input is needed. However, since only the changes in the analyte concentration are determined, a first calibration step may be required at a calibration date t_0 to obtain a reference analyte value AV_(t_0) if the absolute analyte concentration level is to be measured by the analysis apparatus 1.

In another embodiment, represented in figure 9, the principal model may also receive as an input an analyte concentration level AV(t - Δt) at the date t - Δt prior to a given date t in addition to at least one real main sensor datum at the given date t MD_real(t) and the corresponding one or more expected main sensor data MD_pred(t) calculated by a processor implementing the environmental model, and return as an output an analyte concentration level AV(t) at the given date t.

We can see on figure 5 that, in this same extremely simplified case, a principal model is able to predict a blood glucose value varying in the range [70 mg/dL, 320 mg/dL] with a good accuracy. In particular, the mean absolute relative deviation (MARD) is equal to 9,2 % ; the root mean square error is equal to 18,99 mg/dL ; 100% of the points are in zones A and B and 90% are in zone A.

The principal model is estimated with a second training dataset.

The second training dataset can be acquired through one or more measurement campaign.

The second training dataset may be stored on a memory storage device.

The second training dataset may also be formed with the first training dataset as long as this first training dataset comprises several series and the corresponding analyte concentrations to be predicted. It can also be trained on a second dataset.

The principal model can be based on any supervised machine learning technique, such as a random forest, neural networks, etc.

The principal model can also be based on known theoretical relationships between any of the additional parameters and the signal sensed by the main sensor.

The second training dataset may also be divided at this step into two different sets : a learning set and a validation set.

In the case of a glycaemia sensor, if the first and second training datasets can be chosen so that the sensor fits to the Clarke error grid, with no estimated values outside the A and B zones.

Once the environmental model and the principal model are estimated, they can be provided to an only processor or to two different processors.

The process according to the invention can then comprise receiving at least one real main sensor datum at a new date t, at least one real main sensor datum at a date t' prior to date t and at least one or more additional sensor datum at this date t, and calculating an analyte concentration or a quantity in mathematical relationship with an analyte concentration level with the signal processor 13 based on the environmental model and the principal model and the real main sensor datum at date t, the real main sensor datum at the previous date t' and one or more additional data at date t.

One advantage of the process is that the problem is split in two parts, which allows dimensionality reduction for the calculation of the environmental model and the principal model.

As a consequence, each model is easier to estimate and more specialized than a global model of a classical workflow, which implies an improved global accuracy in the case of the invention.

This allows for example to recalculate or adapt one or both of these models for a given patient at sustainable time cost.

By the way, since the output of the environmental model is not the principal analyte concentration (which is not directly correlated with the environmental parameters) but the expected main sensor data (which is directly correlated with these environmental parameters), the environmental model is more likely to be realistic than a global model according to a classical workflow.

In addition to these advantages, the dataset required for the estimation of each model (that is : the first training dataset and the second training dataset) may be globally smaller than the dataset required in a classical workflow due to the dimensionality reduction.

The disclosure also relates to a processor implementing an environmental model receiving as input at least one additional datum at an analysis date t from at least additional sensor configured to measure at least one additional parameter influencing a signal (or a physical phenomenon) sensed by a main sensor configured to sense directly or indirectly a wave being generated in said target in response to an irradiation of a target with a wave emitter and at least one real main sensor datum from the main sensor at a prior date t' earlier than said analysis date t, and providing at least one expected main sensor datum at the analysis date t that estimates at least one datum which would have been obtained with the main sensor at the analysis date t if the analyte concentration level had remained the same at the analysis date t as at the prior date t'.

The disclosure further relates to a processor implementing a principal model receiving at least one expected main sensor datum at an analysis date t from the environmental model and at least one real main sensor datum at the analysis date t from a main sensor configured to sense directly or indirectly a thermal wave being generated in said target in response to an irradiation of a target with a light emitter, and estimating a quantity in mathematical relationship with an analyte concentration level at the analysis date t.

The invention pertains in addition to a computer program comprising instructions which, when the program is executed by a computer, cause the computer to carry out the process according to any of the embodiments described above.

### LIST OF THE REFERENCE SIGNS

1 : portable non-invasive analysis apparatus
11 : light emitter block
12 : detection cell
13 : signal processing module
14 : additional sensor
2 : target

## Claims

1. Process for estimating a quantity in mathematical relationship with an analyte concentration level at an analysis date t in a target, with an analysis apparatus comprising :
- a wave emitter emitting an electromagnetic wave or an acoustic wave,
- a main sensor configured to sense directly or indirectly a wave being generated in said target in response to an irradiation of the target with the wave emitter,
- at least one additional sensor configured to measure at least one additional parameter influencing a signal sensed by the main sensor,
- at least one processor,
the process comprising:
a. providing a set of models comprising :
- an environmental model receiving as input at least one additional datum at an analysis date t from the at least one additional sensor and at least one real main sensor datum from the main sensor at a prior date t' earlier than said analysis date t, and providing at least one expected main sensor datum at the analysis date t that estimates at least one datum which would have been obtained with the main sensor at the analysis date t if the analyte concentration level had remained the same at the analysis date t as at the prior date t',
- and a principal model receiving said at least one expected main sensor datum at the analysis date t from said environmental model and at least one real main sensor datum from the main sensor at the analysis date t, and estimating a quantity in mathematical relationship with an analyte concentration level at the analysis date t ;
b. triggering the irradiation of the target with the wave emitter at a prior date t' earlier than the analysis date t in order to generate a first wave in the target in response to the irradiation, and directly or indirectly detecting this first generated wave with the main sensor in order to acquire at least one real main sensor datum at the prior date t';
c. triggering the irradiation of the target with the wave emitter at the analysis date t in order to generate a second wave in the target in response to the irradiation, directly or indirectly detecting this second generated wave with the main sensor in order to acquire at least one real main sensor datum at the analysis date t, and measuring at least one additional parameter with at least one additional sensor in order to acquire at least one additional datum at the analysis date t with at least one additional sensor ;
d. calculating at least one expected main sensor datum at the analysis date t based on the environmental model, on said at least one additional datum at the analysis date t and on said at least one real main sensor datum at the prior date t' with a processor implementing the environmental model ;
e. calculating with a processor implementing the principal model a quantity in mathematical relationship with an analyte concentration level at the analysis date t based on the principal model, said at least one real main sensor datum at the analysis date t and said calculated at least one expected main sensor datum at the analysis date t.

2. Process for estimating a quantity in mathematical relationship with an analyte concentration level in a target with an analysis apparatus according to claim 1 **characterized in that** the quantity in mathematical relationship with an analyte concentration level at an analysis date t in a target is chosen in the list {analyte concentration level at the analysis date t, variation of the analyte concentration level between a date t' prior to the analysis date t and the analysis date t, rate of change of the analyte concentration level at the analysis date t, a range of analyte concentration levels }.

3. Process for estimating a quantity in mathematical relationship with an analyte concentration level in a target with an analysis apparatus according to claim 1 or claim 2 **characterized in that** the environmental model further receives as an input at least one additional sensor datum at the prior date t' earlier than the analysis date t and **in that** the principal model further receives as input an analyte concentration level at the prior date t'.

4. Process for estimating a quantity in mathematical relationship with an analyte concentration level in a target with an analysis apparatus according to any of claims 1-3 **characterized in that** the wave emitter is a light emitter and the wave generated in the target in response to an irradiation of the target with the light emitter is a thermal wave.

5. Process for estimating a quantity in mathematical relationship with an analyte concentration level in a target with an analysis apparatus according to any of claims 1 to 4 further comprising :
- providing a user interface receiving data from one of said at least one processor ;
- displaying said quantity in mathematical relationship with the analyte concentration level at the analysis date t on the user interface at a display date t+d where d ranges in [0 min ; 30 min].

6. Process for estimating a quantity in mathematical relationship with an analyte concentration level in a target with an analysis apparatus according to any of claims 1 to 5 **characterized in that** the irradiation step c, the calculation step d and the calculation step e are iteratively repeated.

7. Process for estimating a quantity in mathematical relationship with an analyte concentration level in a target with an analysis apparatus according to claim 6 **characterized in that** the process further comprises :
f. calculating with a processor a quantity in mathematical relationship with an analyte concentration level at the analysis date t based on at least two quantities in mathematical relationship with the analyte concentration levels at at least two different analysis dates t1 and t2 calculated at two different calculation steps e, said at least two different analysis dates t1 and t2 being in mathematical relation with the analysis date t.

8. Process for estimating a quantity in mathematical relationship with an analyte concentration level in a target with an analysis apparatus according to any of claims 1-7, comprising prior to the provision of the set of models at step a :
- obtaining a first training dataset with a main sensor and with at least one additional sensor, the first training dataset comprising at least one series of main sensor data and simultaneous additional sensor data, all the main and additional sensor data of a given series being generated in one or more targets having the same analyte concentration level;
- training a first algorithm in a processor with the first training dataset to estimate said environmental model, so that a processor implementing the environmental model can estimate at least one expected main sensor datum at an analysis date t when receiving as input at least one additional sensor datum at said analysis date t and at least one real main sensor datum at a prior date t' earlier than said analysis date t;
- obtaining a second training dataset with a main sensor, at least one additional sensor and a training sensor configured to invasively sense said analyte concentration level and provided non-surgically, the second training dataset comprising at least two series of data, each series comprising at at least two given dates at least one real main sensor datum at each given date, the associated at least one expected main sensor datum at said given date, beforehand calculated with a processor implementing the environmental model, and the quantities in mathematical relationship with the analyte concentration level sensed at said given date with the training sensor, any two given series corresponding to two different analyte concentration levels;
- training a second algorithm in a processor with the second training dataset to estimate said principal model so that a processor implementing the principal model can estimate a quantity in mathematical relationship with an analyte concentration level at an analysis date t when receiving as input said at least one expected main sensor datum at an analysis date t and at least one real main sensor datum at this analysis date t.

9. Process for estimating a quantity in mathematical relationship with an analyte concentration level in a target with an analysis apparatus according to claim 8 **characterized in that** the training of any of the first and second algorithms comprises a supervised machine learning technique.

10. Process for estimating a quantity in mathematical relationship with an analyte concentration level in a target with an analysis apparatus according to claim 8 or claim 9 **characterized in that** the target is a human being and **in that** any of the first and second datasets of the estimation step comprises data typical of a particular group of human beings to which the target belongs.

11. Process for estimating a quantity in mathematical relationship with an analyte concentration level in a target with an analysis apparatus according to any of claims 8 to 10 characterized that the target is a human being and in that any of the first and second datasets of the estimation step comprises data acquired on the target on which the analyzing apparatus is implemented.

12. Process for estimating a quantity in mathematical relationship with an analyte concentration level in a target with an analysis apparatus according to any of claims 1 to 11 **characterized in that** the one or more additional parameters comprise physiological parameters chosen in the list {a temperature of the target, a pressure of the target, a pH of the target, a blood volume parameter, a heart rate, a second analyte concentration level in the target}.

13. Process for estimating a quantity in mathematical relationship with an analyte concentration level in a target with an analysis apparatus according to any of claims 1 to 12 **characterized in that** the one or more additional parameters comprise environmental parameters chosen in the list {a temperature of the environment of the target, a pressure in the environment of the target, a humidity level in the environment of the target}.

14. Analyte monitor (1) for non invasively estimating a quantity in mathematical relationship with an analyte concentration level in a target (2) comprising :
- a wave emitter (11) configured to irradiate the target (2) so that a wave is generated in the target (2) in response to this irradiation;
- a main sensor (12) configured to detect directly or indirectly a wave being generated in a target in response to an irradiation of the target (2);
- at least one additional sensor (14) configured to sense one additional parameter influencing a signal sensed by the main sensor (12);
- at least one data processor module configured to receive at least one main sensor datum and at least one or more additional sensors datum and comprising a first processor adapted to implement an environmental model receiving as input
- at least said one additional datum at an analysis date t at which a quantity in mathematical relationship with an analyte concentration level in a target is to be estimated from said at least one additional sensor configured to measure at least one additional parameter influencing a signal sensed by said main sensor configured to sense directly or indirectly a wave being generated in the target in response to an irradiation of the target with said wave emitter,
- - and at least a real main sensor datum from the main sensor at a prior date t' earlier than said analysis date t,
- and providing at least one expected main sensor datum at the analysis date t that estimates at least one datum which would have been obtained with the main sensor at the analysis date t if the analyte concentration level in the target had remained the same at the analysis date t as at the prior date t',
- and a second processor adapted to implement a principal model receiving said at least one expected main sensor datum at an analysis date t from said first processor and at least one real main sensor datum at the analysis date t from said main sensor configured to sense directly or indirectly a wave being generated in a target in response to an irradiation of the target with said wave emitter, and estimating a quantity in mathematical relationship with an analyte concentration level in the target at the analysis date t.

15. Analyte monitor (1) according to claim 14 **characterized in that** the wave emitter (12) is a light emitter and **in that** the wave generated in the target (2) in response to an irradiation of the target with the light emitter is a thermal wave.

16. Analyte monitor (1) according to any of claims 14 to 15 **characterized in that** the at least one additional sensor is chosen in the list {temperature sensor, pressure sensor, pH-sensor, moisture sensor, humidity sensor, photoplethysmograph, analyte concentration sensor}.

17. Analyte monitor (1) according to any of claims 14 to 16 **characterized in that** the analyte the concentration level with which the quantity to be estimated is in mathematical relationship is blood glucose and **in that** the analyte monitor (1) is a continuous monitor.

18. A computer program comprising instructions which cause the analyte monitor of any of claims 14 to 17 to carry out the process of any of claims 1 to 13.

## Patentansprüche

1. Verfahren zur Abschätzung einer Größe, die in mathematischer Beziehung zu einem Analytkonzentrationsniveau zum Analysezeitpunkt t in einem Ziel steht, mit einem Analysegerät, das Folgendes umfasst:
- ein Wellenemitter, der eine elektromagnetische Welle oder eine akustische Welle aussendet,
- ein Hauptsensor, der dazu eingerichtet ist, direkt oder indirekt eine in dem genannten Ziel erzeugte Welle als Reaktion auf eine Bestrahlung des Ziels mit dem Wellenemitter zu erfassen,
- mindestens ein zusätzlicher Sensor, der dazu eingerichtet ist, mindestens einen zusätzlichen Parameter zu messen, der ein vom Hauptsensor erfasstes Signal beeinflusst,
- mindestens ein Prozessor, wobei das Verfahren Folgendes umfasst:
a. Bereitstellen eines Satzes von Modellen, der Folgendes umfasst:
- ein Umweltmodell, das als Eingabe mindestens einen zusätzlichen Messwert zu einem Analysezeitpunkt t von mindestens einem zusätzlichen Sensor und mindestens einen realen Hauptsensor-Messwert vom Hauptsensor zu einem früheren Zeitpunkt t' vor dem Analysezeitpunkt t erhält, und das mindestens einen erwarteten Hauptsensordatenwert zum Analysezeitpunkt t bereitstellt, der mindestens einen Datenwert schätzt, der mit dem Hauptsensor zum Analysezeitpunkt t erhalten worden wäre, wenn die Analytkonzentration zum Analysezeitpunkt t dieselbe wie zum früheren Zeitpunkt t' gewesen wäre,
- und ein Hauptmodell, das den genannten mindestens einen erwarteten Hauptsensordatenwert zum Analysezeitpunkt t von dem genannten Umweltmodell und mindestens einen tatsächlichen Hauptsensordatenwert vom Hauptsensor zum Analysezeitpunkt t erhält und eine Größe schätzt, die in mathematischer Beziehung zu einem Analytkonzentrationsniveau zum Analysezeitpunkt t steht;
b. Auslösen der Bestrahlung des Ziels mit dem Wellenemitter zu einem früheren Datum t' vor dem Analysezeitpunkt t, um als Reaktion auf die Bestrahlung eine erste Welle im Ziel zu erzeugen, und direktes oder indirektes Erfassen dieser ersten erzeugten Welle mit dem Hauptsensor, um mindestens einen tatsächlichen Hauptsensordatenwert zum früheren Datum t' zu gewinnen;
c. Auslösen der Bestrahlung des Ziels mit dem Wellenemitter zum Analysezeitpunkt t, um als Reaktion auf die Bestrahlung eine zweite Welle im Ziel zu erzeugen, direktes oder indirektes Erfassen dieser zweiten erzeugten Welle mit dem Hauptsensor, um mindestens einen tatsächlichen Hauptsensordatenwert zum Analysezeitpunkt t zu gewinnen, und Messen mindestens eines zusätzlichen Parameters mit mindestens einem zusätzlichen Sensor, um mindestens ein zusätzliches Datum zum Analysezeitpunkt t mit mindestens einem zusätzlichen Sensor zu erlangen;
d. Berechnen mindestens eines erwarteten Hauptsensorwerts zum Analysezeitpunkt t anhand des Umweltmodells, zumindest eines zusätzlichen Werts zum Analysezeitpunkt t und zumindest eines tatsächlichen Hauptsensorwerts zum früheren Zeitpunkt t' mit einem Prozessor, der das Umweltmodell implementiert;
e. Berechnung einer Größe, die in mathematischer Beziehung zu einem Analytkonzentrationsniveau zum Analysezeitpunkt t steht, durch einen Prozessor, der das Hauptmodell implementiert, basierend auf dem Hauptmodell, dem genannten mindestens einen tatsächlichen Hauptsensordatenwert zum Analysezeitpunkt t und dem genannten berechneten mindestens einen erwarteten Hauptsensordatenwert zum Analysezeitpunkt t.

2. Verfahren zur Schätzung einer Größe, die in mathematischer Beziehung zu einem Analytkonzentrationsniveau in einem Ziel steht, mit einem Analysegerät gemäß Anspruch 1 **gekennzeichnet dadurch, dass** die in mathematischer Beziehung zu einem Analytkonzentrationsniveau zum Analysezeitpunkt t in einem Ziel stehende Größe aus der Liste {Analytkonzentrationsniveau zum Analysezeitpunkt t, Änderung des Analytkonzentrationsniveaus zwischen einem Zeitpunkt t' vor dem Analysezeitpunkt t und dem Analysezeitpunkt t, Änderungsrate des Analytkonzentrationsniveaus zum Analysezeitpunkt t, ein Bereich von Analytkonzentrationsniveaus} gewählt wird.

3. Verfahren zur Schätzung einer Größe, die in mathematischer Beziehung zu einem Analytkonzentrationsniveau in einem Ziel steht, mit einem Analysegerät gemäß Anspruch 1 oder Anspruch 2, **gekennzeichnet dadurch, dass** das Umweltmodell zusätzlich als Eingang mindestens einen zusätzlichen Sensordatenwert zum früheren Zeitpunkt t' vor dem Analysezeitpunkt t erhält und dass das Hauptmodell zusätzlich als Eingang ein Analytkonzentrationsniveau zum früheren Zeitpunkt t' erhält.

4. Verfahren zur Schätzung einer Größe, die in mathematischer Beziehung zu einem Analytkonzentrationsniveau in einem Ziel steht, mit einem Analysegerät gemäß einem der Ansprüche 1-3, **dadurch gekennzeichnet, dass** der Wellenemitter ein Lichtemitter ist und die in dem Ziel als Reaktion auf eine Bestrahlung des Ziels mit dem Lichtemitter erzeugte Welle eine thermische Welle ist.

5. Verfahren zur Schätzung einer Größe, die in mathematischer Beziehung zu einem Analytkonzentrationsniveau in einem Ziel steht, mit einem Analysegerät gemäß Ansprüchen 1 bis 4, weiter umfassend:
- Bereitstellen einer Benutzeroberfläche, die Daten von einem der genannten Prozessoren empfängt;
- Anzeigen der besagten Menge in mathematischer Beziehung zur Analytenkonzentration zum Zeitpunkt der Analyse t auf der Benutzeroberfläche zu einem Anzeigedatum t+d, wobei d im Bereich von [0 min; 30 min] liegt.

6. Verfahren zur Schätzung einer Größe, die in mathematischer Beziehung zu einem Analytkonzentrationsniveau in einem Ziel steht, mit einem Analysegerät gemäß einem der Ansprüche 1 bis 5, **dadurch gekennzeichnet, dass** der Bestrahlungsschritt c, der Berechnungsschritt d und der Berechnungsschritt e iterativ wiederholt werden.

7. Verfahren zur Schätzung einer Größe, die in mathematischer Beziehung zu einem Analytkonzentrationsniveau in einem Ziel steht, mit einem Analysegerät gemäß Anspruch 6, **dadurch gekennzeichnet, dass** das Verfahren ferner Folgendes umfasst:
f. Berechnung mittels eines Prozessors einer Größe, die in mathematischer Beziehung zu einem Analytkonzentrationsniveau am Analysezeitpunkt t steht, basierend auf mindestens zwei Größen, die in mathematischer Beziehung zu den Analytkonzentrationsniveaus an mindestens zwei verschiedenen Analysezeitpunkten t1 und t2 stehen und in zwei verschiedenen Berechnungsschritten e berechnet wurden, wobei die genannten mindestens zwei verschiedenen Analysezeitpunkte t1 und t2 in mathematischer Beziehung zum Analysezeitpunkt t stehen.

8. Verfahren zur Schätzung einer Größe, die in mathematischer Beziehung zu einem Analytkonzentrationsniveau in einem Ziel steht, mit einem Analysegerät nach einem der Ansprüche 1-7, umfassend, vor der Bereitstellung der Modellmenge in Schritt a:
- Erhalten eines ersten Trainingsdatensatzes mit einem Hauptsensor und mindestens einem zusätzlichen Sensor, wobei der erste Trainingsdatensatz mindestens eine Serie von Hauptsensordaten und gleichzeitig erhobenen zusätzlichen Sensordaten umfasst, wobei alle Haupt- und zusätzlichen Sensordaten einer gegebenen Serie in einem oder mehreren Zielen mit demselben Analytkonzentrationsniveau erzeugt werden;
- Training eines ersten Algorithmus in einem Prozessor mit dem ersten Trainingsdatensatz zur Schätzung des genannten Umweltmodells, sodass ein Prozessor, der das Umweltmodell implementiert, mindestens einen erwarteten Hauptsensordatenwert zum Analysezeitpunkt t schätzen kann, wenn er als Eingabe mindestens ein zusätzliches Sensordatum zum genannten Analysezeitpunkt t und mindestens einen tatsächlichen Hauptsensordatenwert an einem früheren Zeitpunkt t' vor dem genannten Analysezeitpunkt t erhält;
- Erhalten eines zweiten Trainingsdatensatzes mit einem Hauptsensor, mindestens einem zusätzlichen Sensor und einem Trainingssensor, der so ausgelegt ist, das genannte Analytkonzentrationsniveau invasiv zu erfassen und nicht-chirurgisch bereitgestellt wird, wobei der zweite Trainingsdatensatz mindestens zwei Datenserien umfasst, wobei jede Serie für mindestens zwei vorgegebene Zeitpunkte jeweils mindestens einen tatsächlichen Hauptsensordatenwert an jedem vorgegebenen Zeitpunkt, den zugehörigen mindestens einen zuvor mit einem Prozessor, der das Umweltmodell implementiert, berechneten erwarteten Hauptsensordatenwert, und die mit dem Trainingssensor an dem genannten vorgegebenen Zeitpunkt erfassten Größen in mathematischer Beziehung zum Analytkonzentrationsniveau enthält, wobei zwei beliebige gegebene Serien zwei unterschiedlichen Analytkonzentrationsniveaus entsprechen;
- Training eines zweiten Algorithmus in einem Prozessor mit dem zweiten Trainingsdatensatz zur Schätzung des genannten Hauptmodells, sodass ein Prozessor, der das Hauptmodell implementiert, eineGröße, die in mathematischer Beziehung zu einem Analytkonzentrationsniveau am Analysezeitpunkt t steht, schätzen kann, wenn er als Eingabe den genannten mindestens einen erwarteten Hauptsensordatenwert zum Analysezeitpunkt t und mindestens einen tatsächlichen Hauptsensordatenwert zu diesem Analysezeitpunkt t erhält.

9. Verfahren zur Schätzung einer Größe, die in mathematischer Beziehung zu einem Analytkonzentrationsniveau in einem Ziel mit einem Analysegerät gemäß Anspruch 8, **dadurch gekennzeichnet, dass** das Training eines der ersten und zweiten Algorithmen eine überwachte maschinelle Lerntechnik umfasst.

10. Verfahren zur Schätzung einer Größe, die in mathematischer Beziehung zu einem Analytkonzentrationsniveau in einem Ziel mit einem Analysegerät gemäß Anspruch 8 oder Anspruch 9, **dadurch gekennzeichnet, dass** das Ziel ein Mensch ist und dass einer der ersten und zweiten Datensätze des Schätzungsschritts Daten enthält, die für eine bestimmte Gruppe von Menschen, der das Ziel angehört, typisch sind.

11. Verfahren zur Schätzung einer Größe, die in mathematischer Beziehung zu einem Analytkonzentrationsniveau in einem Ziel mit einem Analysegerät gemäß einem der Ansprüche 8 bis 10, **dadurch gekennzeichnet, dass** das Ziel ein Mensch ist und dass einer der ersten und zweiten Datensätze des Schätzungsschritts Daten enthält, die am Ziel, an dem das Analysegerät eingesetzt ist, erhoben wurden.

12. Verfahren zur Schätzung einer Größe, die in mathematischer Beziehung zu einem Analytkonzentrationsniveau in einem Ziel mit einem Analysegerät gemäß einem der Ansprüche 1 bis 11 **dadurch gekennzeichnet, dass** die einen oder mehrere zusätzlichen Parameter physiologische Parameter aus der Liste {eine Temperatur des Ziels, ein Druck des Ziels, ein pH-Wert des Ziels, ein Blutvolumenparameter, eine Herzfrequenz, ein zweites Analytkonzentrationsniveau im Ziel} umfassen.

13. Verfahren zur Schätzung einer Größe, die in mathematischer Beziehung zu einem Analytkonzentrationsniveau in einem Ziel steht, mit einem Analysegerät gemäß einem der Ansprüche 1 bis 12, **dadurch gekennzeichnet, dass** ein oder mehrere zusätzliche Parameter Umweltparameter aus der Liste {eine Temperatur der Umgebung des Ziels, ein Druck in der Umgebung des Ziels, ein Feuchtigkeitsniveau in der Umgebung des Ziels} umfassen.

14. Analyt-Monitoringgerät (1) zum nicht-invasiven Schätzen einer Größe, die in mathematischer Beziehung zu einem Analytkonzentrationsniveau in einem Ziel (2) steht, umfassend:
- einen Wellensender (11), der so konfiguriert ist, dass er das Ziel (2) bestrahlt, sodass in Reaktion auf diese Bestrahlung eine Welle im Ziel (2) erzeugt wird;
- ein Hauptsensor (12), so ausgelegt, direkt oder indirekt eine im Ziel als Reaktion auf eine Bestrahlung des Ziels (2) erzeugte Welle zu erfassen;
- mindestens ein zusätzlicher Sensor (14), so ausgelegt, einen zusätzlichen Parameter zu erfassen, der ein vom Hauptsensor (12) erfasstes Signal beeinflusst;
- mindestens ein Datenprozessormodul, so ausgelegt, mindestens ein Hauptsensordatum und mindestens ein zusätzliches Sensordatum zu empfangen und einen ersten Prozessor umfassend, der zur Implementierung eines Umweltmodells angepasst ist, das als Eingabe erhält
- mindestens das genannte zusätzliche Datum zum Analysezeitpunkt t, zu dem eine Größe, die in mathematischer Beziehung zu einem Analytkonzentrationsniveau in einem Ziel steht, aus genanntem mindestens einem zusätzlichen Sensor geschätzt werden soll, wobei dieser so ausgelegt ist, mindestens einen zusätzlichen Parameter zu messen, der ein vom genannten Hauptsensor erfasstes Signal beeinflusst, wobei letzterer so ausgelegt ist, direkt oder indirekt eine im Ziel erzeugte Welle zu erfassen, die als Reaktion auf eine Bestrahlung des Ziels mit genanntem Wellenemitter entsteht,
- - und mindestens ein tatsächlicher Hauptsensordatenwert vom Hauptsensor zu einem früheren Zeitpunkt t', der vor dem genannten Analysezeitpunkt t liegt,
- und die Bereitstellung mindestens eines erwarteten Hauptsensordatenwerts zum Analysezeitpunkt t, der mindestens einen Datenwert schätzt, der mit dem Hauptsensor zum Analysezeitpunkt t erhalten worden wäre, wenn das Analytkonzentrationsniveau im Ziel zum Analysezeitpunkt t unverändert gegenüber dem früheren Zeitpunkt t' geblieben wäre,
und einen zweiten Prozessor, der zur Implementierung eines Hauptmodells angepasst ist, das das genannte mindestens einen erwarteten Hauptsensordatenwert zum Analysezeitpunkt t vom genannten ersten Prozessor und mindestens einen tatsächlichen Hauptsensordatenwert zum Analysezeitpunkt t vom genannten Hauptsensor empfängt, wobei letzterer so ausgelegt ist, direkt oder indirekt eine in einem Ziel als Reaktion auf eine Bestrahlung des Ziels mit genanntem Wellenemitter erzeugte Welle zu erfassen, und eine Größe schätzt, die in mathematischer Beziehung zu einem Analytkonzentrationsniveau im Ziel zum Analysezeitpunkt t steht.

15. Analyt-Monitoringgerät (1) nach Anspruch 14, **dadurch gekennzeichnet, dass** der Wellenemitter (12) ein Lichtemitter ist und dass die in dem Ziel (2) als Reaktion auf eine Bestrahlung des Ziels mit dem Lichtemitter erzeugte Welle eine thermische Welle ist.

16. Analyt-Monitoringgerät (1) nach einem der Ansprüche 14 bis 15, **dadurch gekennzeichnet, dass** mindestens ein zusätzlicher Sensor aus der Liste {Temperatursensor, Drucksensor, pH-Sensor, Feuchtesensor, Luftfeuchtigkeitssensor, Photoplethysmograph, Analytkonzentrationssensor} ausgewählt ist.

17. Analyt-Monitoringgerät (1) nach einem der Ansprüche 14 bis 16, **dadurch gekennzeichnet, dass** das Analytkonzentrationsniveau, mit dem die zu schätzende Größe in mathematischer Beziehung steht, Blutglukose ist, und dass das Analyt-Monitoringgerät (1) ein kontinuierlicher Monitor ist.

18. Ein Computerprogramm mit Anweisungen, die das Analyt-Monitoringgerät eines der Ansprüche 14 bis 17 veranlassen, das Verfahren eines der Ansprüche 1 bis 13 durchzuführen.

## Revendications

1. Procédé d'estimation d'une quantité en relation mathématique avec un niveau de concentration d'un analyte à une date d'analyse t dans une cible, avec un appareil d'analyse comprenant :
- un émetteur d'ondes émettant une onde électromagnétique ou une onde acoustique,
- un capteur principal configuré pour détecter directement ou indirectement une onde générée dans ladite cible en réponse à une irradiation de la cible par l'émetteur d'ondes,
- au moins un capteur supplémentaire configuré pour mesurer au moins un paramètre supplémentaire influençant un signal détecté par le capteur principal,
- au moins un processeur,
le procédé comprenant :
a. on fournit un ensemble de modèles comprenant :
- un modèle environnemental recevant en entrée au moins une donnée supplémentaire à une date d'analyse t provenant de l'au moins un capteur supplémentaire et au moins une donnée réelle du capteur principal à une date antérieure t'antérieure à ladite date d'analyse t, et on fournit au moins une donnée attendue du capteur principal à la date d'analyse t qui estime au moins une donnée qui aurait été obtenue avec le capteur principal à la date d'analyse t si le niveau de concentration de l'analyte était resté le même à la date d'analyse t qu'à la date antérieure t',
- et un modèle principal recevant ladite donnée attendue du capteur principal à la date d'analyse t provenant dudit modèle environnemental et au moins une donnée réelle du capteur principal à la date d'analyse t, et estimant une quantité en relation mathématique avec le niveau de concentration de l'analyte à la date d'analyse t ;
b. on déclenche l'irradiation de la cible avec l'émetteur d'ondes à une date antérieure t', antérieure à la date d'analyse t, afin de générer une première onde dans la cible en réponse à l'irradiation, et on détecte directement ou indirectement cette première onde générée avec le capteur principal afin d'acquérir au moins une donnée réelle du capteur principal à la date antérieure t' ;
c. on déclenche l'irradiation de la cible avec l'émetteur d'ondes à la date d'analyse t afin de générer une deuxième onde dans la cible en réponse à l'irradiation, on détecte directement ou indirectement cette deuxième onde générée avec le capteur principal afin d'acquérir au moins une donnée réelle du capteur principal à la date d'analyse t, et on mesure au moins un paramètre supplémentaire avec au moins un capteur supplémentaire afin d'acquérir au moins une donnée supplémentaire à la date d'analyse t avec au moins un capteur supplémentaire ;
d. on calcule au moins une donnée attendue du capteur principal à la date d'analyse t, à partir du modèle environnemental, à partir d'au moins une donnée supplémentaire à la date d'analyse t et à partir d'au moins une donnée réelle du capteur principal à la date antérieure t', à l'aide d'un processeur implémentant le modèle environnemental ;
e. on calcule, à l'aide d'un processeur implémentant le modèle principal, une quantité en relation mathématique avec le niveau de concentration d'un analyte à la date d'analyse t, à partir du modèle principal, de ladite au moins une donnée réelle du capteur principal à la date d'analyse t et de ladite au moins une donnée attendue du capteur principal calculée à la date d'analyse t.

2. Procédé d'estimation d'une grandeur en relation mathématique avec la concentration d'un analyte dans une cible, au moyen d'un appareil d'analyse selon la revendication 1, **caractérisé en ce que** la grandeur en relation mathématique avec la concentration d'un analyte à la date d'analyse t dans une cible est choisie dans la liste suivante : { concentration de l'analyte à la date d'analyse t, variation de la concentration de l'analyte entre une date t' antérieure à la date d'analyse t et la date d'analyse t, taux de variation de la concentration de l'analyte à la date d'analyse t, plage de concentrations de l'analyte}.

3. Procédé d'estimation d'une grandeur en relation mathématique avec la concentration d'un analyte dans une cible, au moyen d'un appareil d'analyse selon la revendication 1 ou 2, **caractérisé en ce que** le modèle environnemental reçoit en outre, en entrée, au moins une donnée de capteur supplémentaire à la date antérieure t', et **en ce que** le modèle principal reçoit en outre, en entrée, la concentration de l'analyte à la date antérieure t'.

4. Procédé d'estimation d'une quantité en relation mathématique avec le niveau de concentration d'un analyte dans une cible à l'aide d'un appareil d'analyse selon l'une quelconque des revendications 1 à 3, **caractérisé en ce que** l'émetteur d'ondes est un émetteur de lumière et que l'onde générée dans la cible en réponse à une irradiation de la cible par l'émetteur de lumière est une onde thermique.

5. Procédé d'estimation d'une grandeur en relation mathématique avec la concentration d'un analyte dans une cible, au moyen d'un appareil d'analyse selon l'une quelconque des revendications 1 à 4, comprenant en outre :
- on fournit une interface utilisateur recevant des données d'un ou plusieurs processeurs ;
- on affiche ladite grandeur en relation mathématique avec la concentration de l'analyte à la date d'analyse t, sur l'interface utilisateur à la date d'affichage t+d, où d est compris dans [0 ; 30 minutes].

6. Procédé d'estimation d'une grandeur en relation mathématique avec la concentration d'un analyte dans une cible, au moyen d'un appareil d'analyse selon l'une quelconque des revendications 1 à 5, **caractérisé en ce que** les étapes d'irradiation c, de calcul d et de calcul e sont répétées de manière itérative.

7. Procédé d'estimation d'une grandeur en relation mathématique avec la concentration d'un analyte dans une cible, au moyen d'un appareil d'analyse selon la revendication 6, **caractérisé en ce que** le procédé comprend en outre :
f. on calcule avec un processeur une quantité en relation mathématique avec un niveau de concentration d'analyte à la date d'analyse t basée sur au moins deux quantités en relation mathématique avec les niveaux de concentration d'analyte à au moins deux dates d'analyse différentes t1 et t2 calculées à deux étapes de calcul e différentes, lesdites au moins deux dates d'analyse différentes t1 et t2 étant en relation mathématique avec la date d'analyse t.

8. Procédé d'estimation d'une grandeur en relation mathématique avec le niveau de concentration d'un analyte dans une cible, au moyen d'un appareil d'analyse selon l'une quelconque des revendications 1 à 7, comprenant, avant la fourniture de l'ensemble de modèles à l'étape a :
- on obtient un premier jeu de données d'apprentissage avec un capteur principal et au moins un capteur additionnel, ce premier jeu de données d'apprentissage comprenant au moins une série de données du capteur principal et des données simultanées du capteur additionnel, toutes les données des capteurs principal et additionnel d'une série donnée étant générées dans une ou plusieurs cibles présentant le même niveau de concentration d'analyte ;
- on entraîne un premier algorithme dans un processeur à l'aide du premier jeu de données d'apprentissage afin d'estimer ledit modèle environnemental, de sorte qu'un processeur implémentant le modèle environnemental puisse estimer au moins une donnée attendue du capteur principal à une date d'analyse t, lorsqu'il reçoit en entrée au moins une donnée de capteur additionnel à ladite date d'analyse t et au moins une donnée réelle du capteur principal à une date antérieure t' antérieure à ladite date d'analyse t ;
- on obtient un second jeu de données d'entraînement avec un capteur principal, au moins un capteur supplémentaire et un capteur d'entraînement configuré pour mesurer de manière invasive le niveau de concentration de l'analyte et fourni sans intervention chirurgicale, ce second jeu de données d'entraînement comprenant au moins deux séries de données, chaque série comprenant, à au moins deux dates données, au moins une donnée réelle du capteur principal, la donnée attendue du capteur principal correspondante, calculée au préalable par un processeur implémentant le modèle environnemental, et les quantités en relation mathématique avec le niveau de concentration de l'analyte mesuré par le capteur d'entraînement à chaque date, deux séries quelconques correspondant à deux niveaux de concentration d'analyte différents ;
- on entraîne un second algorithme sur un processeur à l'aide du second jeu de données d'entraînement afin d'estimer le modèle principal de sorte qu'un processeur implémentant le modèle principal peut estimer une quantité en relation mathématique avec le niveau de concentration de l'analyte à une date d'analyse t, à partir de la donnée attendue du capteur principal et de la donnée réelle du capteur principal à cette même date d'analyse t.

9. Procédé d'estimation d'une grandeur en relation mathématique avec la concentration d'un analyte dans une cible, au moyen d'un appareil d'analyse selon la revendication 8, **caractérisé en ce que** l'apprentissage de l'un quelconque des premier et deuxième algorithmes repose sur une technique d'apprentissage automatique supervisé.

10. Procédé d'estimation d'une grandeur en relation mathématique avec la concentration d'un analyte dans une cible, au moyen d'un appareil d'analyse selon la revendication 8 ou 9, **caractérisé en ce que** la cible est un être humain et **en ce que** l'un quelconque des premier et deuxième ensembles de données de l'étape d'estimation comprend des données typiques d'un groupe particulier d'êtres humains auquel appartient la cible.

11. Procédé d'estimation d'une grandeur en relation mathématique avec la concentration d'un analyte dans une cible, au moyen d'un appareil d'analyse selon l'une quelconque des revendications 8 à 10, **caractérisé en ce que** la cible est un être humain et **en ce que** l'un quelconque des premier et deuxième ensembles de données de l'étape d'estimation comprend des données acquises sur la cible sur laquelle l'appareil d'analyse est mis en œuvre.

12. Procédé d'estimation d'une grandeur en relation mathématique avec la concentration d'un analyte dans une cible, au moyen d'un appareil d'analyse selon l'une quelconque des revendications 1 à 11, **caractérisé en ce que** le ou les paramètres supplémentaires comprennent des paramètres physiologiques choisis parmi la liste suivante : {température de la cible, pression de la cible, pH de la cible, volume sanguin, fréquence cardiaque, concentration d'un second analyte dans la cible}.

13. Procédé d'estimation d'une grandeur en relation mathématique avec la concentration d'un analyte dans une cible, au moyen d'un appareil d'analyse selon l'une quelconque des revendications 1 à 12, **caractérisé en ce que** le ou les paramètres supplémentaires comprennent des paramètres environnementaux choisis parmi la liste suivante : {température de l'environnement de la cible, pression de l'environnement de la cible, taux d'humidité de l'environnement de la cible}.

14. Dispositif de surveillance d'analyte (1) pour l'estimation non invasive d'une grandeur en relation mathématique avec le niveau de concentration d'un analyte dans une cible (2), comprenant :
- un émetteur d'ondes (11) configuré pour irradier la cible (2) de manière à générer une onde dans celle-ci en réponse à cette irradiation ;
- un capteur principal (12) configuré pour détecter, directement ou indirectement, l'onde générée dans la cible en réponse à son irradiation (2) ;
- au moins un capteur supplémentaire (14) configuré pour détecter un paramètre supplémentaire influençant le signal détecté par le capteur principal (12).
- au moins un module de traitement de données configuré pour recevoir au moins une donnée de capteur principal et au moins une donnée de capteur supplémentaire, et comprenant un premier processeur adapté à la mise en œuvre d'un modèle environnemental recevant en entrée
- au moins ladite donnée supplémentaire à une date d'analyse t, à laquelle une quantité en relation mathématique avec le niveau de concentration d'un analyte dans une cible doit être estimée à partir dudit au moins un capteur supplémentaire configuré pour mesurer au moins un paramètre supplémentaire influençant un signal détecté par ledit capteur principal configuré pour détecter directement ou indirectement une onde générée dans la cible en réponse à l'irradiation de celle-ci par ledit émetteur d'ondes,
- - et au moins une donnée réelle du capteur principal à une date antérieure t', antérieure à ladite date d'analyse t,
- et fournissant au moins une donnée attendue du capteur principal à la date d'analyse t, qui estime au moins une donnée qui aurait été obtenue avec le capteur principal à la date d'analyse t si le niveau de concentration d'un analyte dans la cible était resté le même à la date d'analyse t qu'à la date antérieure t',
et un deuxième processeur adapté pour mettre en œuvre un modèle principal recevant au moins une donnée attendue de capteur principal à une date d'analyse t provenant dudit premier processeur et au moins une donnée réelle de capteur principal à la date d'analyse t provenant dudit capteur principal configuré pour détecter directement ou indirectement une onde générée dans une cible en réponse à une irradiation de la cible avec ledit émetteur d'ondes, et pour estimer une quantité en relation mathématique avec un niveau de concentration d'analyte dans la cible à la date d'analyse t.

15. Dispositif de surveillance d'analyte (1) selon la revendication 14, **caractérisé en ce que** l'émetteur d'ondes (12) est un émetteur de lumière et **en ce que** l'onde générée dans la cible (2) en réponse à l'irradiation de la cible par l'émetteur de lumière est une onde thermique.

16. Dispositif de surveillance d'analyte (1) selon l'une quelconque des revendications 14 à 15, **caractérisé en ce que** l'au moins un capteur supplémentaire est choisi parmi la liste suivante : capteur de température, capteur de pression, capteur de pH, capteur d'humidité, capteur d'humidité relative, photopléthysmographe, capteur de concentration d'analyte.

17. Dispositif de surveillance d'analyte (1) selon l'une quelconque des revendications 14 à 16, **caractérisé en ce que** l'analyte dont le niveau de concentration sert de relation mathématique à la quantité à estimer est la glycémie, et **en ce que** le dispositif de surveillance d'analyte (1) est un dispositif de surveillance continue.

18. Programme informatique comprenant des instructions permettant au dispositif de surveillance d'analyte selon l'une quelconque des revendications 14 à 17 d'exécuter le procédé selon l'une quelconque des revendications 1 à 13.
